# EUROPEAN PATENT SPECIFICATION

(11) **EP 2 837 629 B1**
(45) Date of publication and mention of the grant of the patent: **22.03.2017**
(21) Application number: 12874086.7
(22) Date of filing: 27.12.2012
(51) Int. Cl.: C07C 49/17

(54) **METHOD FOR SYNTHESIZING SAPROPTERIN DIHYDROCHLORIDE**
VERFAHREN ZUR SYNTHESE VON SAPROPTERINDIHYDROCHLORID
PROCÉDÉ DE SYNTHÈSE DE DICHLORHYDRATE DE SAPROPTÉRINE

(30) Priority: 10.04.2012 CN 201210102879
(43) Date of publication of application: 18.02.2015
(73) Proprietor: Asymchem Laboratories (Tianjin) Co., Ltd, Tianjin 300457 (CN); Asymchem Life Science (Tianjin) Co., Ltd, Tianjin 300457 (CN); Tianjin Asymchem Pharmaceutical Co., Ltd, Tianjin 300000 (CN); Asymchem Laboratories (Fuxin) Co., Ltd, Fuxin, Liaoning 123000 (CN); Jilin Asymchem Laboratories Co., Ltd, Dunhua, Jilin 133700 (CN)
(72) Inventor: HONG, Hao, Tianjin 300457 (CN); GAGE, James, Tianjin 300457 (CN); CHEN, Chaoyong, Tianjin 300457 (CN); LU, Jiangping, Tianjin 300457 (CN); ZHOU, Yan, Tianjin 300457 (CN); LIU, Shuangyong, Tianjin 300457 (CN)
(74) Representative: Grünecker Patent- und Rechtsanwälte PartG mbB
(86) International application number: PCT/CN2012/087732
(87) International publication number: WO 2013/152608

(56) References cited:
- EP-A2- 0 153 696
- EP-B1- 0 191 335
- WO-A1-2005/063752
- WO-A1-2009/047902
- WO-A1-2009/088979
- WO-A2-2005/049614
- CN-A- 102 633 799
- JP-A- S 574 990
- JP-A- S60 204 786
- JP-B2- 2 575 781
- US-A1- 2006 142 573

## Description

### Technical field of the invention

The present invention relates to a method for synthesizing a medicine for treating Phenylketonuria (PKU), and particularly to a method for synthesizing sapropterin dihydrochloride.

### Backpround of the invention

Sapropterin dihydrochloride, chemical name (6R)-2-amino-6-[(1R,2S)-1,2-dihydroxypropyl]-5,6,7,8-tetrahydro-4(1H)-pteridinone dihydrochloride, molecular formula C₉H₁₅N₅O₃·2HCl, and CAS registry number 69056-38-28, is a synthetic product of tetrahydrobiopterin (BH₄) dihydrochloride. BH4 is a cofactor of Phenylalanine Hydroxylase (PAH). Tyrosine is acquired from Phenylalanine (Phe) through hydroxylation under the action of PAH which is low in activity or even inactive in PKU patients, while BH₄ is able to activate PAH, promote normal oxidative metabolism of Phe in the bodies of the patients, and reduce the Phe levels in the bodies of some patients. On December 16th, 2007, the sapropterin dihydrochloride tablets produced by BioMarin Pharmaceutical Inc. in USA were approved by the Food and Drug Administration (FDA) for marketing for treatment of PKU. Because of the effective activity of sapropterin dihydrochloride, it is extremely necessary to select a route applicable to industrial production with high product purity.

At present, BH₄ is mainly synthesized by the following methods reported in literatures:
1. Preparation using 4-hydroxy-2,5,6-triaminopyrimidine (ATP) and 5-deoxy-L-arabinose as raw materials, please see literature E.L.Patterson et al., J.Am.Chem.Soc.78, 5868 (1956).
2. Preparation using TAP and 5-deoxy-L-arabinose phenylhydrazone as raw materials, please see literature Matsuura et al., Bull.Chem.Soc.Jpn., 48,3767 (1975);
3. Preparation by reaction of raw materials hydroxyl-protected TAP and 4-acetyl-2,3-epoxypentanal through oxidation of iodine and a dehydroxylation protecting group, please see literature Matsuura et al., Chemistry of Organic Synthesis,Ml/g.46.No.6,P570 (1988).

These traditional methods for preparing BH4 have the following major disadvantages: raw materials are expensive, arabinose which can be hardly acquired is used as a carbon atom radical for asymmetric synthesis; there are multiple steps in reactions with low yield, and low product purity, 5-deoxy-L-arabinose is easily degraded in a reaction solution, and products of the synthesis routes above have low stereoselectivity. To sum up, the traditional synthesis methods are not applicable to mass industrial production. Therefore, a synthesis route, which is applicable to industrial production with high product purity, high yield and high stereoselectivity, needs to be searched urgently.
WO 2009/047902 A1 discloses a method which enables to commercially produce a pteridine compound with high yield. Specifically, it discloses a method for producing a pteridine compound represented by the general formula (3) 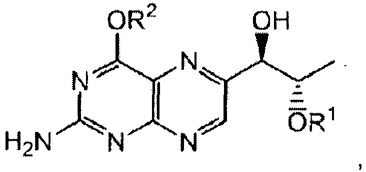 which is characterized in that an optically active epoxy aldehyde compound represented by the general formula (1) 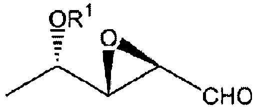 and a pyrimidine compound represented by the general formula (2) 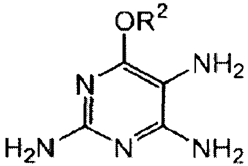 are condensed in a polar solvent in the presence of an acid having a pKa of not more than 4.5, and then the resulting compound is subjected to oxidation.
WO 2005/049614 A2 discloses a process for the preparation of tetrahydrobiopterin from neoprerin and/or 6-substituted pterins with an improved yield and a high stereoselectivity. Also disclosed are novel individual intermediates useful for the preparation of tetrahydrobiopterin, such as selectively protected neopterin.
JP 2575781 B discloses production of L-bioprerin. To obtain the title compound, useful as a precursor of (6R)-tetrahydrobiopterin (remedy for Parkinson's disease), from an inexpensive raw material in good yield, an alkyl (S)-lactate is used as a starting raw material, passing through several novel intermediates.

### Summary of the invention

The present invention provides a method for synthesizing a sapropterin dihydrochloride compound. The present invention reduces a synthesis route of sapropterin dihydrochloride, and resolves a racemate intermediate or an intermediate having a low antimer isomerism value by using a chiral resolving reagent, thereby obtaining an intermediate having a high antimer isomerism value. Raw materials are cheap and readily available, and the cost is significantly reduced, hence providing an effective scheme for mass industrial production of sapropterin dihydrochloride.

The present invention provides a method for synthesizing sapropterin dihydrochloride, characterized in that it comprises the following steps:
Step 1: a compound 1 of the formula 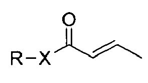 is subjected to epoxidation to generate a compound 2 of the formula 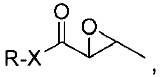 wherein X=NH or O, R=C1-C6 alkane or benzyl;
Step 2: in the presence of acetone, a Lewis acid, an inorganic base liquid, the compound 2 of the formula 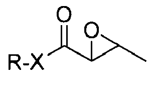 reacts to generate a compound 3 of the formula 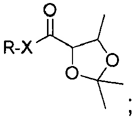
Step 3: the compound 3 of the formula 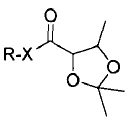 reacts in an alkaline solution, a polar solvent is used to dissolve a filter cake obtained through centrifugation, then a resolving reagent is added to perform resolution to obtain a compound 4 of the formula 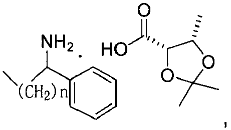 wherein n=0,1;
Step 4: dissolving the compound 4 of the formula 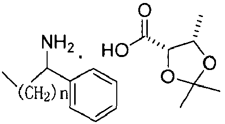 in an ether solvent, and performing separation in acidic conditions to obtain an organic phase, and adding N,N-diisopropylethylamine to the organic phase to obtain a compound 5 of the formula 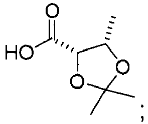
Step 5: using the compound 5 of the formula 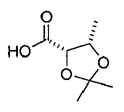 as a raw material to synthesize a compound 6 of the formula 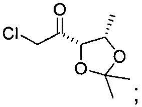
Step 6: reacting the compound 6 of the formula 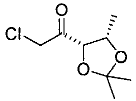 with a trinitride to generate a compound 7 of the formula 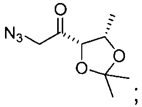
Step 7: subjecting the compound 7 of the formula 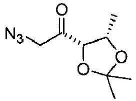 to hydrogenation to obtain a compound 8 of the formula 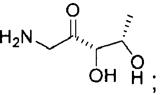
Step 8: reacting the compound 8 of the formula 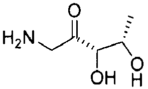 and a compound A of the formula 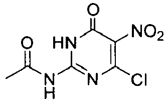 to generate a compound 9 of the formula 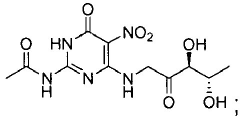
Step 9: subjecting the compound 9 of the formula 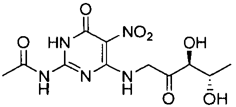 to cyclization to obtain a compound 10 of the formula 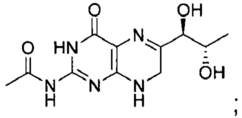
Step 10: adding a catalyst to the compound 10 of the formula 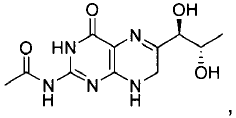 introducing hydrogen to perform a reaction, and then perform quenching in hydrochloric acid having a concentration of 10% to 20% to obtain sapropterin dihydrochloride.

Also provided is a method for preparing (3S,4S)-1-amino-3,4-dihydroxy-2-pentanone, characterized in that it comprises the following steps:
Step 1: using a compound 5 of the formula 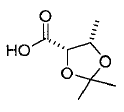 as a raw material to synthesize a compound 6 of the formula 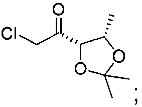
Step 2: reacting the compound 6 of the formula 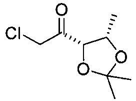 with a trinitride to generate a compound 7 of the formula 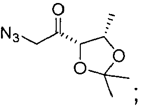
Step 3: subjecting the compound 7 of the formula 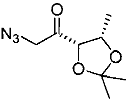 to hydrogenation to obtain a compound 8 of the formula

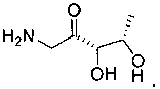

Preferred embodiments of the present invention are set forth in the dependent claims.

The present invention has the following advantages: 1. raw materials applied by the synthesis method are readily available, and the cost is significantly reduced compared with the prior art; 2. the route of the present invention is simple, thus greatly reducing a synthesis route of sapropterin dihydrochloride; 3. technological conditions are stable, the whole operation process is simple with less discharge of waste water, waste gas, and waste residues, and less pollution, hence providing an effective scheme for mass industrial production of sapropterin dihydrochloride; 4. the present invention, which resolves a racemate intermediate or an intermediate having a low antimer isomerism value by using a chiral resolving reagent to obtain an intermediate having a high antimer isomerism value, is a supplement to a chiral route; 5. the present invention can obtain a target product with a purity higher than 98% and an enantiomeric excess as high as more than 98%.

### Brief description of the drawings

The accompanying drawings of the specification are used for providing further understanding to the present invention and constitute a part of the present invention. The exemplary embodiments of the present invention and the illustrations thereof are used for explaining the present invention, instead of constituting an improper limitation to the present invention. In the accompanying drawings:

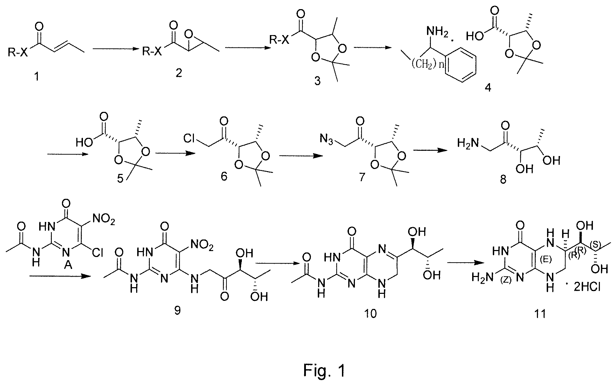

Fig. 1 is a flowchart of a chiral preparation process of a sapropterin dihydrochloride compound involved in the present invention.

### Detailed description of the invention

It should be noted that, if there is no conflict, the embodiments in the present invention and the characteristics in the embodiments can be combined with one another. The present invention will be described in details below with reference to the accompanying drawings and in combination with the embodiments.

The ranges in the embodiments are caused by certain fluctuation of the temperatures and pH values as reactions progress in an experiment.

Embodiment 1: main raw material: 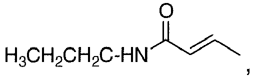 R=-CH₂CH₂CH₃ and X=NH
Step 1: add 950L(10g/ml) of pure water, and 95kg(1 eq) of crotonyl propylamine 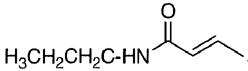 to a 2000L reaction kettle, increase the system temperature to 40±5°C, add 208kg(1.5eq) of N-bromobutanimide, react for 3 hours while preserving the temperature, add 300kg(1.5eq) of a sodium hydroxide solution having a concentration of 15% to the system, react for 3.5 hours while preserving the temperature, perform extraction and concentration to obtain 67.6kg of a compound 2,3 epoxy-butyryl propylamine, with a yield of 63%;
Step 2: in the presence of 219 kg of (8eq) acetone, add 25kg(0.4eq) of aluminium chloride to a 2000L reaction kettle, control the temperature at 20±5°C, add 67.6kg(1.0eq) of 2,3 epoxy-butyryl propylamine 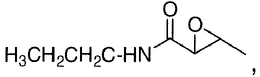 react for 8 hours while preserving the temperature, add 939kg of a sodium carbonate (1.5eq) solution having a concentration of 8% to the system, and perform liquid separation, extraction, and concentration in the system to obtain 75.1 kg of 2,3-acetonide-propylbutyramide 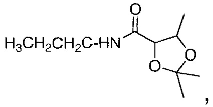 with a yield of 79%;
Step 3: add 450.6(6ml/g) of tetrahydrofuran, and 75.1 kg(1 eq) of 2,3-acetonide-propylbutyramide 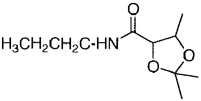 to a 1000L reaction kettle, increase the temperature to 30±5°C, add 11.3kg(1.2eq) of pure water and 117.2kg(1.2eq) of a methanol solution of sodium methoxide having a concentration of 29%, react for 6 hours while preserving the temperature, perform centrifugation, dissolve a filter cake in 525.7L (7 ml/g) of tetrahydrofuran, add 127.1 kg(2eq) of L-α-phenylethylamine, preserve the temperature at 22±5°C for 4 hours, and perform centrifugation and drying to obtain 27.3kg of 1-phenylethanamine 2,2,5-trimethyl-1,3-dioxolane-4-carboxylate 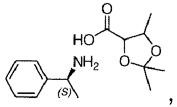 with a yield of 26%;
Step 4: add 28L(5ml/g) of 2-methyltetrahydrofuran, and 5.6kg (1eq) of 1-phenylethanamine 2,2,5-trimethyl-1,3-dioxolane-4-carboxylate 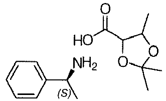 to a 72 L reaction bottle, then add a dilute hydrochloric acid aqueous solution having a concentration of 8% to the system to regulate the pH at 2±0.5, control the temperature at 0±5°C, react for 1 hour while preserving the temperature, perform liquid separation to obtain an organic phase, add 4.5kg of (1 eq) N,N-diisopropylethylamine to the organic phase, and concentrate the system to obtain 3.0kg of (4S,5S)-2,2,5-trimethyl-1,3-dioxolan-4-methanoic acid 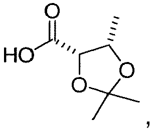 with a yield of 95%;
Step 5: add 30 L (10 ml/g) of 2-methyltetrahydrofuran, 3.0 kg of (4S,5S)-2,2,5-trimethyl-1,3-dioxolan-4-methanoic acid 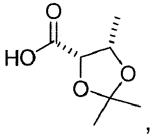 and 4.3kg(2eq) of N,N-diisopropylethylamine to a 72L reaction bottle, reduce the temperature to -20±5°C, add 2.7kg(1.3eq) of ethyl chloroformate, react for 1.5 hours while preserving the temperature, introduce a diazomethane gas for 1.5 hours, add 10.3kg (3eq) of a hydrochloride ethanol solution having a concentration of 20%, react for 1.5 hours, add triethylamine to regulate the pH value to 8±0.5, and perform extraction, liquid separation and concentration to obtain 3.1 kg of (4S,5S)-2,2,5-trimethyl-5-chloroacetyl-1,3-dioxolane 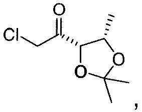 with a yield of 85%;
Step 6: add 31L(10ml/g) of acetone, 3.1 kg of (4S,5S)-2,2,5-trimethyl-5-chloroacetyl-1,3-dioxolane 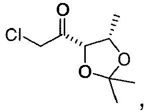 1.9kg(1.8eq) of sodium azide, and 0.5kg (0.2eq) of sodium iodide to a 72L bottle, react the system for 25 hours while preserving the temperature at 30±5°C, perform filtering and concentration to obtain an acetone solution containing 3.05kg of (4S,5S)-2,2,5-trimethyl-5-(2-azidoacetyl)-1,3-dioxolane 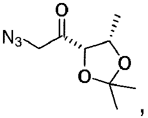 with a yield of 95%;
Step 7: add 30.5L (10ml/g) of tetrahydrofuran, 4.4kg(1.1eq) of triphenylphosphine, and 0.3kg (1.1 eq) of water to a 100L reaction kettle, regulate the pH of the system to 3±0.5 with citric acid, add the acetone solution containing 3.05kg (1 eq) of (4S,5S)-2,2,5-trimethyl-5-(2-azidoacetyl)-1,3-dioxolane 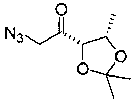 preserve the temperature at 20±5°C, react for 8 hours, perform suction filtration and concentration to obtain a filtrate containing 1.8kg of (3S,4S)-1-amino-3,4-dihydroxy-2-pentanone 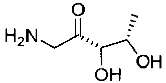 which is directly used in the next step, with a yield of 90%;
Step 8: add 18.9 L(9 ml/g) of isopropanol, 2.3 L (1.1ml/g ) of pure water, 0.1 kg of (0.1 eq) of sodium iodide, 1.76kg(1.1 eq) of compound A (2-amino-6-chloro-5-nitro-3H-pyrimidin-4-one), 0.92kg(1eq) of (3S,4S)-1-amino-3,4-dihydroxy-2-pentanone 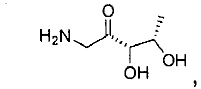 and 3.5kg(5eq) of triethylamine to a 50L reaction bottle, react the system for 6 hours while preserving the temperature at 50±5°C, then add a potassium dihydrogen phosphate-dipotassium hydrogen phosphate aqueous solution to regulate the pH of the system to 7±0.5; and filter the system to obtain 1.02kg of 2-acetylamino-5-nitro-6-((3S,4S)-3,3-dihydroxy-2-oxo-pentylamino)-pyrimidin-4-one 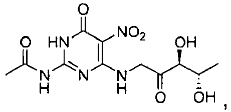 with a yield of 45%;
Step 9: add 2.0kg(1eq) of 2-acetylamino-5-nitro-6-((3S,4S)-3,3-dihydroxy-2-oxo-pentylamino)-pyrimidin-4-one 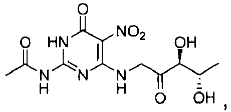 70L(35ml/g) of pure water and 0.6kg(0.3g/g) of Raney nickel to a 100L autoclave, introduce hydrogen until the pressure of the reaction system is 0.6±0.05MPa, control the temperature of the system at 20±5°C and the pressure at 0.6±0.05MPa, react for 20 hours, filter the system, and regulate the pH to 11.5±0.5 to obtain of an aqueous solution containing 1.7kg of acetylamino-7,8-dihydropteridine 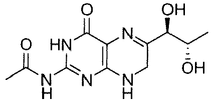 which is used directly in the next step;
Step 10: add 0.255kg(0.15g/g) of 20% palladium on carbon to the aqueous solution containing 1.7kg of acetylamino-7,8-dihydropteridine 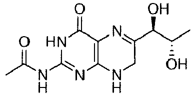 obtained in Step 9, introduce hydrogen until the pressure of the reaction kettle is 0.6±0.05MPa, control the temperature of the system at 20±5°C and the pressure at 0.6±0.05MPa, react for 80 hours, after reacting thoroughly, perform quenching in 10.29kg(7eq) of dilute hydrochloric acid having a concentration of 15%, and perform suction filtration and drying to the system to obtain a target product, i.e. a sapropterin dihydrochloride crude product of 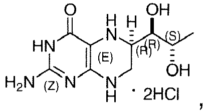 recrystallize and purify the crude product by 25L (14.7ml/g) of methanol at 20±5°C to obtain 0.95kg of a pure product, with a yield of 50%, a purity of 98.5% and an enantiomeric excess of 99.2%.

Embodiment 2: main raw material 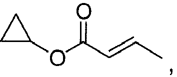 R= 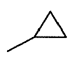 and X=O
Step 1: add 2016L(20g/ml) of methanol, and 100.8kg(1eq) of crotonate cyclopropylalkyl ester 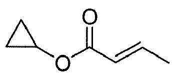 to a 3000 L reaction kettle, increase the system temperature to 50±5°C, add 414kg(3eq) of meta-chloroperoxybenzoic acid, react for 5 hours while preserving the temperature, add 673kg(3eq) of a potassium hydroxide solution having a concentration of 20% to the system, react for 4 hours while preserving the temperature, perform extraction and concentration to obtain 69.4kg of a compound 2,3 epoxy-cyclopropylalkyl butyrater 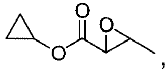 with a yield of 61%;
Step 2: in the presence of 425kg of (15eq) acetone, add 79.2kg(1 eq) of ferric chloride to a 2000L reaction kettle, control the temperature at 30±5°C, add 69.4kg(1.0eq) of 2,3 epoxy- cyclopropylalkyl butyrate 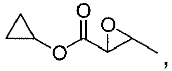 react for 10 hours while preserving the temperature, add 1552kg of a sodium carbonate (3eq) solution having a concentration of 10% to the system, and perform liquid separation, extraction, and concentration in the system to obtain 75.3kg of 2,3-acetonide-cyclopropylalkyl butyrate 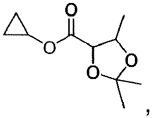 with a yield of 77%;
Step 3: add 753(10ml/g) of methanol, and 75.3kg(1 eq) of 2,3-acetonide-cyclopropylalkyl butyrate 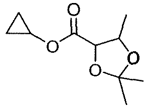 to a 1000L reaction kettle, increase the temperature to 40±5°C, add 20.2kg(3eq) of pure water and 210kg(2eq) of a potassium hydroxide solution having a concentration of 20%, react for 8 hours while preserving the temperature, perform centrifugation, dissolve a filter cake in 753L (10ml/g) of methanol, add 322kg(5eq) of L-α-amphetamine, preserve the temperature at 30±5°C for 5 hours, and perform centrifugation and drying to obtain 27.2kg of 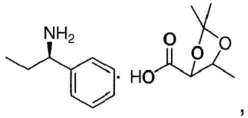 with a yield of 24.5%;
Step 4: add 27 L (10 ml/g) of tetrahydrofuran, 2.7 kg (1eq) of (4S,5S)-2,2,5-trimethyl-1,3-dioxolan-4-phenylpropylamino carboxylate 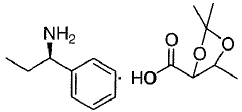 to a 72L reaction bottle, then add a dilute hydrochloric acid aqueous solution having a concentration of 10% to the system to regulate the pH at 3±0.5, control the temperature at 10±5°C, react for 1 hour, perform liquid separation to obtain an organic phase, add 6.1 kg of (3eq) N,N-diisopropylethylamine to the organic phase, and concentrate the system to obtain 1.3kg of (4S,5S)-2,2,5-trimethyl-1,3-dioxolan-4-methanoic acid 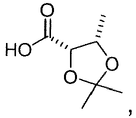 with a yield of 90%;
Step 5: add 20L(15 ml/g) of tetrahydrofuran, 1.3kg of (4S,5S)-2,2,5-trimethyl-1,3-dioxolan-4-methanoic acid 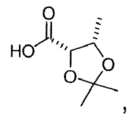 and 8kg(5eq) of N,N-diisopropylethylamine to a 72L reaction bottle, reduce the temperature to 0±5°C, add 2.9kg(3eq) of propyl chloroformate, react for 1 to 2 hours while preserving the temperature, introduce a diazomethane gas for 2 hours, add 12.7kg (5eq) of a hydrochloride ethanol solution having a concentration of 20%, react for 2 hours, add sodium carbonate to regulate the pH value to 9±0.5, and perform extraction, liquid separation and concentration to obtain 1.3kg of (4S,5S)-2,2,5-trimethyl-5-chloroacetyl-1,3-dioxolane 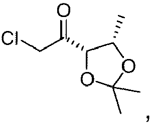 with a yield of 82%;
Step 6: add 19.5L(15ml/g) of acetonitrile, 1.3kg of (4S,5S)-2,2,5-trimethyl-5-chloroacetyl-1,3-dioxolane 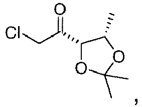 3.1 kg(4eq) of azidotrimethylsilane, and 0.8kg (0.8eq) of sodium iodide to a 72L bottle, react the system for 30 hours while preserving the temperature at 40±5°C, perform filtering and concentration to obtain an acetonitrile solution containing 1.21 kg of 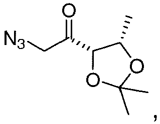 with a yield of 90%;
Step 7: add 18.2 L (15 ml/g) of 1,4-dioxane and 0.73 kg (0.6 g/g) of Raney nickel to a 50 L reaction kettle, introduce hydrogen until the system pressure is 0.9±0.1MPa, regulate the pH of the system to 1±0.5 with concentrated hydrochloric acid, add the acetonitrile solution containing 1.21 kg(1 eq) of (4S,5S)-2,2,5-trimethyl-5-(2-azidoacetyl)-1,3-dioxolane 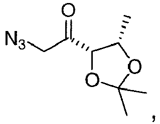 react at 30±5°C for 8 hours, perform suction filtration and concentration to obtain 0.71kg of (3S,4S)-1-amino-3,4-dihydroxy-2-pentanone 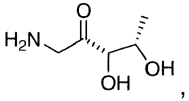 with a yield of 87.5%;
Step 8: add 47.5 L (15 ml/g) of methanol, 15.8L(5ml/g ) of pure water, 1.28kg of (0.5eq) of potassium iodide, 3.6kg(1.5eq) of compound A (2-amino-6-chloro-5-nitro-3H-pyrimidin-4-one), 1.4kg(1eq) of (3S,4S)-1-amino-3,4-dihydroxy-2-pentanone 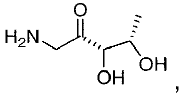 and 6.4 kg (8eq) of pyridine to a 100 L reaction bottle, react the system for 8 hours while preserving the temperature at 80±5°C, then add an ammonium formate-ammonia aqueous solution to regulate the pH of the system to 8±0.5; and filter the system to obtain 1.47kg of 2-acetylamino-5-nitro-6-((3S,4S)-3,3-dihydroxy-2-oxo-pentylamino)-pyrimidin-4-one 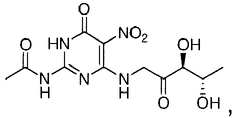 with a yield of 43.2%;
Step 9: add 2.94kg(1 eq) of 2-acetylamino-5-nitro-6-((3S,4S)-3,3-dihydroxy-2-oxo-pentylamino)-pyrimidin-4-one 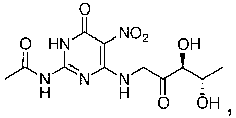 147L(50ml/g) of methanol and 1.76kg(0.6g/g) of 5% palladium on carbon to a 200 L autoclave, introduce hydrogen until the pressure of the system is 0.9±0.05 MPa, control the temperature of the system at 30±5°C and the pressure at 0.9±0.05 MPa, react for 24 hours, filter the system, and regulate the pH to 12±0.5 to obtain a methanol solution containing 2.5kg of acetylamino-7,8-dihydropteridine 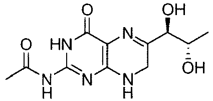 which is used directly in the next step;
Step 10: add 1.5 kg (0.6 g/g) of Raney nickel to the methanol solution containing 2.5kg of acetylamino-7,8-dihydropteridine 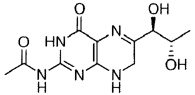 obtained in Step 9, introduce hydrogen until the pressure of the system is 0.9±0.05MPa, control the temperature of the system at 30±5°C and the pressure at 0.9±0.05MPa, react for 84 hours, after reacting thoroughly, perform quenching in 16.2kg(10eq) of dilute hydrochloric acid having a concentration of 20%, and perform suction filtration and drying to the system to obtain a target product, i.e. a crude product of sapropterin dihydrochloride 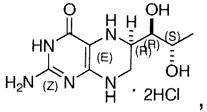 recrystallize and purify the crude product by 62.5L (25ml/g) of acetone at 40±5°C to obtain 1.31 kg of a pure product, with a yield of 47%, a purity of 98.1% and an enantiomeric excess of 98.9%.

Embodiment 3: main raw material: 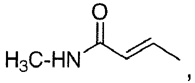 R=-CH₃ and X=NH
Step 1: add 495L(5g/ml) of ethanol, and 99kg(1 eq) of crotonyl methanamine 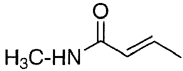 to a 2000L reaction kettle, increase the system temperature to 35±5°C, add 180.2kg(1eq) of a tert-butyl hydroperoxide toluene solution having a concentration of 50%, react for 2 hours while preserving the temperature, add 400kg(1 eq) of a sodium hydroxide solution having a concentration of 10% to the system, react for 3 hours while preserving the temperature, perform extraction and concentration to obtain 70.2kg of 2,3 epoxy - butyryl methylamine 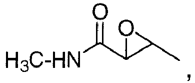 with a yield of 61%;
Step 2: in the presence of 106kg of (3eq) acetone, add 2.6kg(0.1 eq) of lithium chloride to a 1000L reaction kettle, control the temperature at 10±5°C, add 70.2kg(1.0eq) of 2,3 epoxy - butyryl methylamine, react for 5 hours while preserving the temperature, add 610kg of a potassium bicarbonate (0.5eq) solution having a concentration of 5% to the system, and perform liquid separation, extraction, and concentration in the system to obtain 81.3kg of 2,3-acetonide-alkylformamide 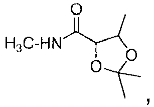 with a yield of 77%;
Step 3: add 243.9 L-(3ml/g) of ethanol, and 81.3kg(1 eq) of 2,3-acetonide-alkylformamide 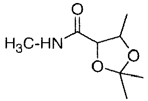 to a 1000L reaction kettle, increase the temperature to 250±5°C, add 4.23kg(0.5eq) of pure water and 47kg(0.5eq) of a sodium hydroxide solution having a concentration of 20%, react for 3 hours while preserving the temperature, perform centrifugation, dissolve a filter cake in 122.6L (2ml/g) of ethanol, add 56.9kg(1 eq) of L-α-phenylethylamine, preserve the temperature at 15±5°C for 3 hours, and perform centrifugation and drying to obtain 32.3kg of (4S,5S)-2,2,5-trimethyl-1,3-dioxolan-4-phenylpropylamino carboxylate 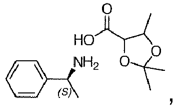 with a yield of 24.5%;
Step 4: add 30L(3ml/g) of 1,4-dioxane, 10kg (1eq) of (4S,5S)-2,2,5-trimethyl-1,3-dioxolan-4-phenylpropylamino carboxylate 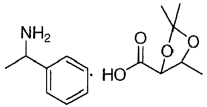 to a 72L reaction bottle, then add a dilute phosphoric acid aqueous solution having a concentration of 5% to the system to regulate the pH at 1±0.5, control the temperature at -10±5°C, react for 1 hour, perform liquid separation to obtain an organic phase, add 3.3kg of (0.8eq) N,N-diisopropylethylamine to the organic phase, and concentrate the system to obtain 5.2kg of (4S,5S)-2,2,5-trimethyl-1,3-dioxolan-4-methanoic acid 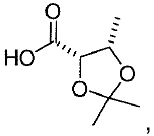 with a yield of 91%;
Step 5: add 26 L (5 ml/g) of 1,4-dioxane, 5.2 kg (1 eq) of (4S,5S)-2,2,5-trimethyl-1,3-dioxolan-4-methanoic acid 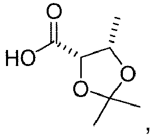 and 3.7kg(1 eq) of N,N-diisopropylethylamine to a 72L reaction bottle, reduce the temperature to -30±5°C, add 3.1 kg(1 eq) of methyl chloroformate, react for 1 hour while preserving the temperature, introduce a diazomethane gas for 1 hour, add 2kg (1eq) of a hydrochloride ethanol solution having a concentration of 20%, react for 1 hour, add potassium bicarbonate to regulate the pH value to 7±0.5, and perform extraction, liquid separation and concentration to obtain 5kg of (4S,5S)-2,2,5-trimethyl-5-chloroacetyl-1,3-dioxolane 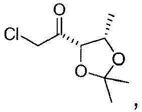 with a yield of 81%;
Step 6: add 25L(5ml/g) of methanol, 5kg of (4S,5S)-2,2,5-trimethyl-5-chloroacetyl-1,3-dioxolane 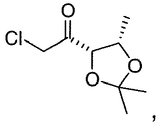 1.7 kg (1 eq) of sodium azide, and 0.22 kg (0.05eq) of potassium iodide to a 72 L bottle, after react the system for 20 hours while preserving the temperature at 15±5°C, perform filtering and concentration to obtain a methanol solution containing 4.5kg of (4S,5S)-2,2,5-trimethyl-5-(2-azidoacetyl)-1,3-dioxolane 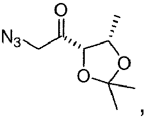 with a yield of 87%;
Step 7: add 22.5 L (5 ml/g) of methyl tert-butyl ether and 0.3 kg (0.05g/g) of 10% palladium on carbon to a 100L reaction kettle, introduce hydrogen until the system pressure is 0.4±0.1 MPa, regulate the pH of the system to 4±0.5 with benzenesulfonic acid, add the methanol solution containing 4.5kg (1 eq) of (4S,5S)-2,2,5-trimethyl-5-(2-azidoacetyl)-1,3-dioxolane 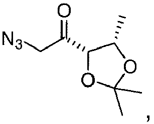 preserve the temperature at 10±5°C, react for 5 hours, perform suction filtration and concentration to obtain a filtrate containing 2.6kg of (3S,4S)-1-amino-3,4-dihydroxy-2-pentanone a yield of 86%; 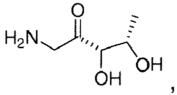 with
Step 8: add 21 L (5 ml/g) of ethanol, 4.2L(1ml/g ) of pure water, 0.1 kg of (0.05eq) sodium iodide, 3.2kg(1 eq) of compound A (2-amino-6-chloro-5-nitro-3H-pyrimidin-4-one), 1.83kg(1eq) of (3S,4S)-1-amino-3,4-dihydroxy-2-pentanone 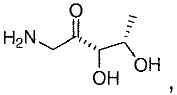 and 4.4kg(3eq) of sodium carbonate to a 50L reaction bottle, react the system for 4 hours while preserving the temperature at 30±5°C, then add a sodium dihydrogen phosphate-disodium hydrogen phosphate aqueous solution to regulate the pH of the system to 6±0.5; and filter the system to obtain 1.9kg of 2-acetylamino-5-nitro-6-((3S,4S)-3,3-dihydroxy-2-oxo-pentylamino)-pyrimidin-4-one 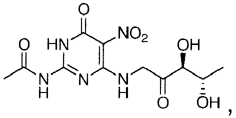 with a yield of 42%;
Step 9: add 3.8kg(1 eq) of 2-acetylamino-5-nitro-6-((3S,4S)-3,3-dihydroxy-2-oxo-pentylamino)-pyrimidin-4-one 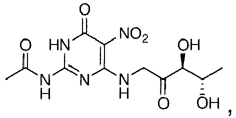 76L(20ml/g) of ethanol and 0.2kg(0.05g/g) of 20% palladium on carbon to a 100L autoclave, introduce hydrogen until the system pressure is 0.4±0.05MPa, control the temperature of the system at 15±5°C and the pressure at 0.4±0.05MPa, react for 18 hours, filter the system, and regulate the pH to 11±0.5 to obtain of an ethanol solution containing 3.25kg of acetylamino-7,8-dihydropteridine 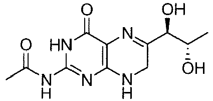 which is used directly in the next step;
Step 10: add 0.16kg(0.05g/g) of platinum dioxide in the presence of the ethanol solution containing 3.25kg of acetylamino-7,8-dihydropteridine 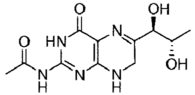 obtained in Step 9, introduce hydrogen until the system pressure is 0.4±0.05MPa, control the temperature of the system at 10±5°C and the pressure at 0.4±0.05MPa, react for 72 hours, after reacting thoroughly, perform quenching in 12.6kg(3eq) of dilute hydrochloric acid having a concentration of 10%, and perform suction filtration and drying to the system to obtain a target product, i.e. a crude product of sapropterin dihydrochloride 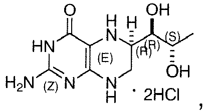 recrystallize and purify the crude product by 16.3L (5ml/g) of isopropanol at 0±5°C, to obtain 1.52kg of a pure product, with a yield of 42%, a purity of 98.0% and an enantiomeric excess of 98.7%.

Embodiment 4: main raw material: 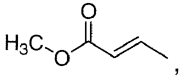 R=-CH₃ and X=O
Step 1: add 900 L (15 g/ml) of isopropanol, and 60 kg (1 eq) of methyl crotonate 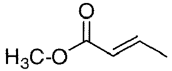 to a 2000 L reaction kettle, increase the system temperature to 45+5°C, add 178kg(2.5eq) of N-bromobutanimide, react for 3.5 hours while preserving the temperature, add 420kg(2.5eq) of a potassium hydroxide solution having a concentration of 20% to the system, react for 3.5 hours while preserving the temperature, perform extraction and concentration to obtain 42.6kg of 2,3 epoxy-methyl butyrate 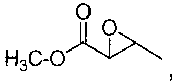 with a yield of 61.2%;
Step 2: in the presence of 256 kg of (12eq) acetone, add 40 kg (0.8eq) of zinc chloride to a 2000 L reaction kettle, control the temperature at 25±5°C, add 42.6kg(1.0eq) of 2,3 epoxy-methyl butyrate 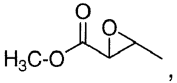 react for 9 hours while preserving the temperature, add 1408kg of a potassium carbonate (2.5eq) solution having a concentration of 9% to the system, and perform liquid separation, extraction, and concentration in the system to obtain 49.8kg of 2,3-acetonide-methyl butyrate 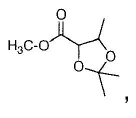 with a yield of 78%;
Step 3: add 398(8ml/g) of methanol, and 49.8kg(1 eq) of 2,3-acetonide-methyl butyrate 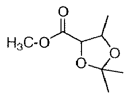 to a 1000L reaction kettle, increase the temperature to 35±5°C, add 9.3kg(1.8eq) of pure water and 144.5kg(1.8eq) of a potassium hydroxide aqueous solution having a concentration of 20%, react for 6.5 hours while preserving the temperature, perform centrifugation, dissolve a filter cake in 398L (8ml/g) of methanol, add 154.7kg(4eq) of L-α-amphetamine, preserve the temperature at 25±5°C for 4.5 hours, and perform centrifugation and drying to obtain 21.1 kg of 1-phenylpropan-1-amine (4S,5S)-2,2,5-trimethyl-1,3-dioxolane-4-carboxylate 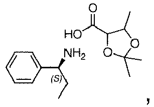 with a yield of 25%;
Step 4: add 48 L (8 ml/g) of methyl tert-butyl ether, 6kg (1 eq) of 1-phenylpropan-1-amine (4S,5S)-2,2,5-trimethyl-1,3-dioxolane-4-carboxylate 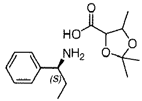 to a 72L reaction bottle, then add a dilute hydrochloric acid aqueous solution having a concentration of 9% to the system to regulate the pH at 2.5±0.5, control the temperature at -5±5°C, react for 1 hour, perform liquid separation to obtain an organic phase, add 6.6kg of (2.5eq) N,N-diisopropylethylamine to the organic phase, and concentrate the system to obtain 3.0kg of 1,3-dioxolan-4-carboxylic acid 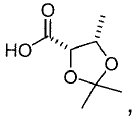 with a yield of 92%;
Step 5: add 36L(12 ml/g) of tetrahydrofuran, 3.0kg of (4S,5S)-2,2,5-trimethyl-1,3-dioxolan-4-methanoic acid 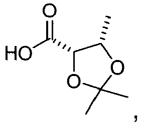 and 9.7kg(4eq) of N,N-diisopropylethylamine to a 72L reaction bottle, reduce the temperature to -25±5°C, add 4.4kg(2.5eq) of methyl chloroformate, react for 1.5 hours while preserving the temperature, introduce a diazomethane gas for 1.5 hours, add 15.3kg (4.5eq) of a hydrochloride ethanol solution having a concentration of 20%, react for 1.5 hours, add triethylamine to regulate the pH value to 8.5±0.5, and perform extraction, liquid separation and concentration to obtain 3.0kg of (4S,5S)-2,2,5-trimethyl-5-chloroacetyl-1,3-dioxolane 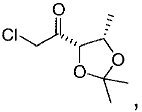 with a yield of 83%;
Step 6: add 36L(12ml/g) of acetone, 3kg of (4S,5S)-2,2,5-trimethyl-5-chloroacetyl-1,3-dioxolane 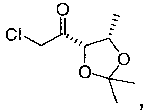 3kg(3eq) of sodium azide, and 1.5kg (0.6eq) of sodium iodide to a 72L bottle, react the system for 27 hours while preserving the temperature at 32±5°C, perform filtering and concentration to obtain an acetone solution containing 2.8kg of (4S,5S)-2,2,5-trimethyl-5-(2-azidoacetyl)-1,3-dioxolane 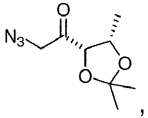 with a yield of 91%;
Step 7: add 33.7L (12ml/g) of 2-methyltetrahydrofuran, 8.6kg(2.0eq) of triphenylphosphine, and 0.5kg(2.0eq) of water to a 72L reaction kettle, regulate the pH of the system to 3±0.5 with acetic acid, add the acetone solution containing 2.8kg of (4S,5S)-2,2,5-trimethyl-5-(2-azidoacetyl)-1,3-dioxolane 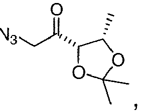 preserve the temperature at 25±5°C, react for 8.5 hours, perform suction filtration and concentration to obtain a filtrate containing 1.6kg of (3S,4S)-1-amino-3,4-dihydroxy-2-pentanone 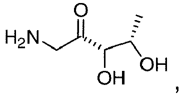 with a yield of 87.5%;
Step 8: add 19.7 L(12 ml/g) of methanol, 6.4L(4ml/g ) of pure water, 0.8kg of (0.4eq) of sodium iodide, 4.0kg(1.4eq) of compound A (2-amino-6-chloro-5-nitro-3H-pyrimidin-4-one), 1.6kg(1eq) of (3S,4S)-1-amino-3,4-dihydroxy-2-pentanone 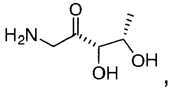 and 8.7kg(7eq) of potassium bicarbonate to a 50L reaction bottle, react the system for 7 hours while preserving the temperature at 70±5°C, then add a sodium dihydrogen phosphate-disodium hydrogen phosphate aqueous solution to regulate the pH of the system to 7.5±0.5; and filter the system to obtain 1.7kg of 2-acetylamino-5-nitro-6-((3S,4S)-3,3-dihydroxy-2-oxo-pentylamino)-pyrimidin-4-one 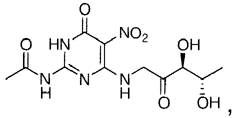 with a yield of 43%;
Step 9: add 1.7kg(1 eq) of 2-acetylamino-5-nitro-6-((3S,4S)-3,3-dihydroxy-2-oxo-pentylamino)-pyrimidin-4-one 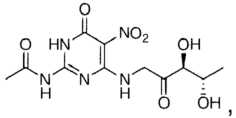 78.7L(45ml/g) of methanol and 0.9kg(0.5g/g) of 5% palladium on carbon to a 100L autoclave, introduce hydrogen until the reaction system pressure is 0.8±0.05MPa, control the temperature of the system at 25±5°C and the pressure at 0.8±0.05MPa, react for 22 hours, filter the system, and regulate the pH to 11±0.5 to obtain a methanol solution containing 1.5kg of acetylamino-7,8-dihydropteridine 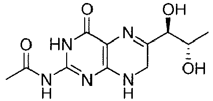 which is used directly in the next step;
Step 10: add 0.7kg(0.05g/g) of 5% palladium on carbon in the presence of the methanol solution containing 1.5kg of acetylamino-7,8-dihydropteridine 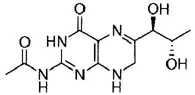 obtained in Step 9, introduce hydrogen until the pressure of the reaction kettle is 0.8±0.05MPa, control the temperature of the system at 25±5°C and the pressure at 0.8±0.05MPa, react for 82 hours, after reacting thoroughly, perform quenching in 31.9kg(9eq) of dilute hydrochloric acid having a concentration of 15%, and perform suction filtration and drying to the system to obtain a target product, i.e. a crude product of sapropterin dihydrochloride 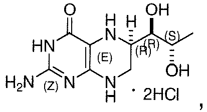 recrystallize and purify the crude product by 29L (20ml/g) of methanol at 35±5°C to obtain 0.8 kg of a pure product, with a yield of 45%, a purity of 98.3% and an enantiomeric excess of 99.1%.

Embodiment 5: main raw material: 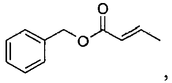 R= 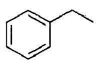 and X=O
Step 1: add 780L(12g/ml) of ethanol, and 65kg(1eq) of benzyl crotonate 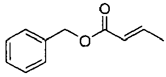 to a 2000L reaction kettle, increase the system temperature to 42±5°C, add 127.3kg(2eq) of meta-chloroperoxybenzoic acid, react for 3 hours while preserving the temperature, add 147.6kg(2eq) of a sodium hydroxide solution having a concentration of 20% to the system, react for 3.2 hours while preserving the temperature, perform extraction and concentration to obtain 44kg of 2,3-epoxy-benzyl butyrate 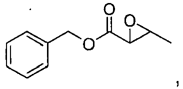 with a yield of 62%;
Step 2: in the presence of 132.8kg of (10eq) acetone, add 5.8kg(0.6eq) of lithium chloride to a 1000L reaction kettle, control the temperature at 22±5°C, add 44kg(1.0eq) of 2,3-epoxy-benzyl butyrate 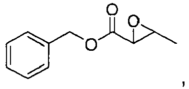 react for 7 hours while preserving the temperature, add 343kg of a potassium dicarbonate (1.5eq) solution having a concentration of 10% to the system, and perform liquid separation, extraction, and concentration in the system to obtain 44kg of 2,3-acetonide-benzyl butyrate 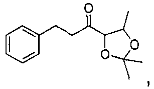 with a yield of 77.5%;
Step 3: add 352(8ml/g) of ethanol, and 44kg(1 eq) of 2,3-acetonide-benzyl butyrate 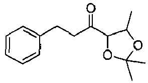 to a 1000L reaction kettle, increase the temperature to 37±5°C, add 4.8kg(1.5eq) of pure water and 53.2kg(1.5eq) of a sodium hydroxide aqueous solution having a concentration of 20%, react for 6 hours while preserving the temperature, perform centrifugation, dissolve a filter cake in 352L (8ml/g) of ethanol, add 71.9kg(3eq) of L-α-amphetamine, preserve the temperature at 22±5°C for 4 hours, and perform centrifugation and drying to obtain 13.2kg of 1-phenylpropan-1-amine (4S,5S)-2,2,5-trimethyl-1,3-dioxolane-4-carboxylate 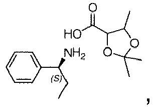 with a yield of 25.3%;
Step 4: add 48L(6ml/g) of 1,4-dioxane, 8kg (1 eq) of 1-phenylpropan-1-amine (4S,5S)-2,2,5-trimethyl-1,3-dioxolane-4-carboxylate 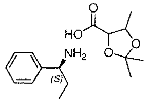 to a 72 L reaction bottle, then add a dilute sulphuric acid aqueous solution having a concentration of 10% to the system to regulate the pH at 2.5±0.5, control the temperature at -5±5°C, react for 1 hour, perform liquid separation to obtain an organic phase, add 7.0kg of (2.0eq) N,N-diisopropylethylamine to the organic phase, and concentrate the system to obtain 4.1 kg of (4S,5S)-2,2,5-trimethyl-1,3-dioxolan-4-methanoic acid 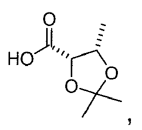 with a yield of 93.5%;
Step 5: add 49L(12 ml/g) of 2-methyltetrahydrofuran, 4.1kg of 1,3-dioxolan-4-carboxylic acid 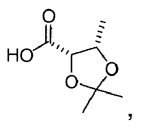 and 13.1 kg(4eq) of N,N-diisopropylethylamine to a 100L reaction bottle, reduce the temperature to -22±5°C, add 5.5kg(2.0eq) of ethyl chloroformate, react for 1.8 hours while preserving the temperature, introduce a diazomethane gas for 1.8 hours, add 18.5kg (4.5eq) of a hydrochloride ethanol solution having a concentration of 20%, react for 1.8 hours, add potassium bicarbonate to regulate the pH value to 8.5±0.5, and perform extraction, liquid separation and concentration to obtain 4.1 kg of (4S,5S)-2,2,5-trimethyl-5-chloroacetyl-1,3-dioxolane 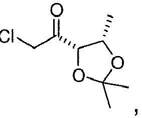 with a yield of 83.7%;
Step 6: add 49L(12ml/g) of acetone, 4.1 kg of (4S,5S)-2,2,5-trimethyl-5-chloroacetyl-1,3-dioxolane 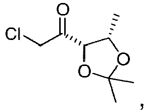 3.4kg(2.5eq) of sodium azide, and 1.8kg (0.5eq) of potassium iodide to a 72L bottle, react the system for 26 hours while preserving the temperature at 34±5°C, perform filtering and concentration to obtain an acetone solution containing 3.9kg of (4S,5S)-2,2,5-trimethyl-5-(2-azidoacetyl)-1,3-dioxolane 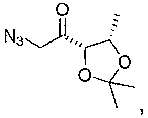 with a yield of 91.5%;
Step 7: add 46.4L (12ml/g) of methyl tert-butyl ether and 1.2kg(0.3g/g) of Raney nickel to a 100L reaction kettle, introduce hydrogen until the system pressure is 0.8±0.1 MPa, regulate the pH of the system to 3±0.5 with concentrated sulfuric acid, add an acetonitrile solution containing 3.9kg(1 eq) of (4S,5S)-2,2,5-trimethyl-5-(2-azidoacetyl)-1,3-dioxolane 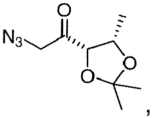 react at 27±5°C for 8.5 hours, perform suction filtration and concentration to obtain 2.3kg of (3S,4S)-1-amino-3,4-dihydroxy-2-pentanone 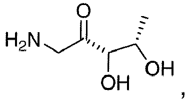 with a yield of 89%;
Step 8: add 23L(10ml/g) of propanol, 6.9L(3ml/g) of pure water, 0.9kg of (0.3eq) of potassium iodide, 4.8kg(1.2eq) of compound A (2-amino-6-chloro-5-nitro-3H-pyrimidin-4-one), 2.3kg(1eq) of (3S,4S)-1-amino-3,4-dihydroxy-2-pentanone 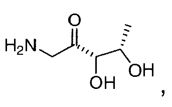 and 10.5kg(6eq) of diisopropylamine to a 50L reaction bottle, react the system for 7 hours while preserving the temperature at 72±5°C, then add a potassium dihydrogen phosphate-dipotassium phosphate aqueous solution to regulate the pH of the system to 7.5±0.5; and filter the system to obtain 2.5kg of 2-acetylamino-5-nitro-6-((3S,4S)-3,3-dihydroxy-2-oxo-pentylamino)-pyrimidin-4-one 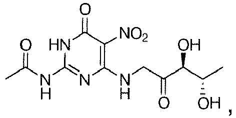 with a yield of 44%;
Step 9: add 1.25kg(1 eq) of 2-acetylamino-5-nitro-6-((3S,4S)-3,3-dihydroxy-2-oxo-pentylamino)-pyrimidin-4-one 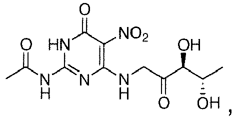 50L(40ml/g) of ethanol and 0.5kg(0.4g/g) of 10% palladium on carbon to a 100L autoclave, introduce hydrogen until the reaction system pressure is 0.8±0.05MPa, control the temperature of the system at 27±5°C and the pressure at 0.8±0.05MPa, react for 24 hours, filter the system, and regulate the pH to 11±0.5 to obtain an ethanol solution containing 1.1kg of acetylamino-7,8-dihydropteridine 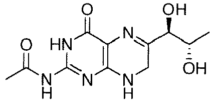 which is used directly in the next step;
Step 10: add 0.44kg(0.4g/g) of 10% palladium on carbon in the presence of the ethanol solution containing 1.1 kg of acetylamino-7,8-dihydropteridine 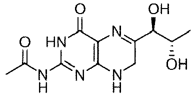 obtained in Step 9, introduce hydrogen until the pressure of the reaction kettle is 0.8±0.05MPa, control the temperature of the system at 25±5°C and the pressure at 0.8±0.05MPa, react for 80 hours, after reacting thoroughly, perform quenching in 20kg(8eq) of dilute hydrochloric acid having a concentration of 15%, and perform suction filtration and drying to the system to obtain a target product, i.e. a crude product of sapropterin dihydrochloride 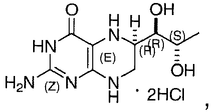 recrystallize and purify the crude product by 21.4L (20ml/g) of ethanol at 35±5°C to obtain 0.4 kg of a pure product, with a yield of 46.2%, a purity of 98.5% and an enantiomeric excess of 99.2%.

Embodiment 6: main raw material: 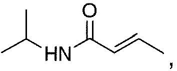 R= 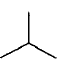 and X=N
Step 1: add 510L(6g/ml) of methanol, and 85kg(1eq) of (E)-N-isopropylbut-2-enamide 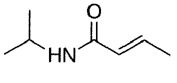 to a 2000 L reaction kettle, increase the system temperature to 37±5°C, add 120.5kg(1 eq) of tert-butyl hydroperoxide toluene solution having a concentration of 50%, react for 2.5 hours while preserving the temperature, add 133.7kg(1 eq) of a sodium hydroxide solution having a concentration of 20% to the system, react for 3.5 hours while preserving the temperature, perform extraction and concentration to obtain 59kg of N-isopropyl-3-methyloxirane-2-carboxamide 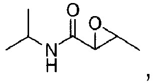 with a yield of 61.7%;
Step 2: in the presence of 119.8kg of (5eq) acetone, add 9.3kg(0.1 eq) of zinc bromide to a 1000L reaction kettle, control the temperature at 15±5°C, add 59kg(1.0eq) of N-isopropyl-3-methyloxirane-2-carboxamide 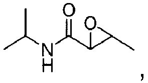 react for 6 hours while preserving the temperature, add 173kg of a sodium dicarbonate (0.5eq) solution having a concentration of 10% to the system, and perform liquid separation, extraction, and concentration in the system to obtain 64.7kg of N-isopropyl-2,2,5-trimethyl-1,3-dioxolane-4-carboxamide 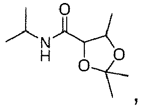 with a yield of 78%;
Step 3: add 259(4ml/g) of tetrahydrofuran, and 64.7kg(1 eq) of N-isopropyl-2,2,5-trimethyl-1,3-dioxolane-4-carboxamide 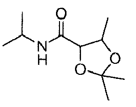 to a 1000 L reaction kettle, increase the temperature to 27±5°C, add 2.9kg(0.5eq) of pure water and 29.9kg(0.5eq) of a methanol solution of sodium methoxide having a concentration of 29%, react for 4 hours while preserving the temperature, perform centrifugation, dissolve a filter cake in 194L (3ml/g) of tetrahydrofuran, add 39kg(1 eq) of L-α-phenylethylamine, preserve the temperature at 18±5°C for 3.5 hours, and perform centrifugation and drying to obtain 22.4kg of 1-phenylethanamine 2,2,5-trimethyl-1,3-dioxolane-4-carboxylate 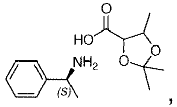 with a yield of 24.7%;
Step 4: add 30L(3ml/g) of 2-methyltetrahydrofuran, 10kg (1eq) of 1-phenyltehanamine -2,2,5-trimethyl-1,3-dioxolane-4-carboxylat 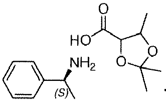 to a 72L reaction bottle, then add a dilute phosphoric acid aqueous solution having a concentration of 10% to the system to regulate the pH at 1.5±0.5, control the temperature at -5±5°C, react for 1 hour, perform liquid separation to obtain an organic phase, add 3.7kg of (0.8eq) N,N-diisopropylethylamine to the organic phase, and concentrate the system to obtain 5.3kg of (4S,5S)-2,2,5-trimethyl-1,3-dioxolan-4-methanoic acid 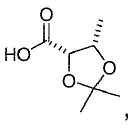 with a yield of 92.5%;
Step 5: add 42L(8 ml/g) of 1,4-dioxane, 5.3kg of 1,3-dioxolan-4-carboxylic acid 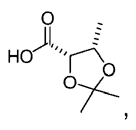 and 8.5kg(2eq) of N,N-diisopropylethylamine to a 100L reaction bottle, reduce the temperature to -10±5°C, add 4kg(21.0eq) of propyl chloroformate, react for 2 hours while preserving the temperature, introduce a diazomethane gas for 2 hours, add 12kg (2eq) of a hydrochloride ethanol solution having a concentration of 20%, react for 2 hours, add sodium hydroxide to regulate the pH value to 7.5±0.5, and perform extraction, liquid separation and concentration to obtain 5.1 kg of (4S,5S)-2,2,5-trimethyl-5-chloroacetyl-1,3-dioxolane 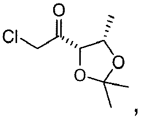 with a yield of 81%;
Step 6: add 41 L(8ml/g) of tetrahydrofuran, 5.1kg of (4S,5S)-2,2,5-trimethyl-5-chloroacetyl-1,3-dioxolane 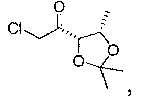 3.1 kg(1 eq) of azidotrimethylsilane, and 0.5kg (0.1 eq) of sodium iodide to a 72L bottle, react the system for 30 hours while preserving the temperature at 12±5°C, perform filtering and concentration to obtain an acetone solution containing 4.6kg of (4S,5S)-2,2,5-trimethyl-5-(2-azidoacetyl)-1,3-dioxolane 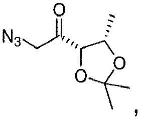 with a yield of 87.5%;
Step 7: add 28L (6ml/g) of 1,4-dioxane and 0.23kg(0.05g/g) of 10% palladium on carbon to a 50L reaction kettle, introduce hydrogen until the system pressure is 0.8±0.1MPa, regulate the pH of the system to 3±0.5 with acetic acid, add an acetonitrile solution containing 4.6kg(1 eq) of (4S,5S)-2,2,5-trimethyl-5-(2-azidoacetyl)-1,3-dioxolane 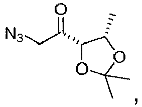 react at 27±5°C for 8.5 hours, perform suction filtration and concentration to obtain 2.7 kg of (3S,4S)-1-amino-3,4-dihydroxy-2-pentanone 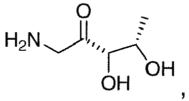 with a yield of 87.7%;
Step 8: add 16.3 L (6 ml/g) of isopropanol, 2.7 L (1 g/g ) of pure water, 0.4kg of (0.1 eq) of sodium iodide, 4.8kg(1.0eq) of compound A (2-amino-6-chloro-5-nitro-3H-pyrimidin-4-one), 2.7kg(1eq) of (3S,4S)-1-amino-3,4-dihydroxy-2-pentanone 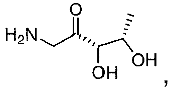 and 8.7kg(4eq) of sodium carbonate to a 50L reaction bottle, react the system for 7 hours while preserving the temperature at 45±5°C, then add an ammonium formate-ammonia aqueous solution to regulate the pH of the system to 6.5±0.5; and filter the system to obtain 2.85kg of 2-acetylamino-5-nitro-6-((3S,4S)-3,3-dihydroxy-2-oxo-pentylamino)-pyrimidin-4-one 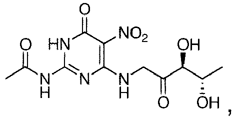 with a yield of 42.5%;
Step 9: add 2kg(1 eq) of 2-acetylamino-5-nitro-6-((3S,4S)-3,3-dihydroxy-2-oxo-pentylamino)-pyrimidin-4-one 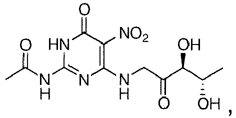 60L(30ml/g) of ethanol and 0.2kg(0.1g/g) of platinum dioxide to a 100L autoclave, introduce hydrogen until the reaction system pressure is 0.6±0.05MPa, control the temperature of the system at 20±5°C and the pressure at 0.6±0.05MPa, react for 20 hours, filter the system, and regulate the pH to 11±0.5 to obtain an ethanol solution containing 1.7kg of acetylamino-7,8-dihydropteridine 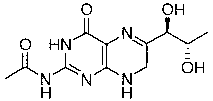 which is used directly in the next step;
Step 10: add 0.2kg(0.1g/g) of platinum dioxide in the presence of the ethanol solution containing 1.7kg of acetylamino-7,8-dihydropteridine 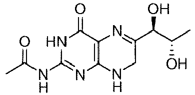 obtained in Step 9, introduce hydrogen until the pressure of the reaction kettle is 0.6±0.05MPa, control the temperature of the system at 15±5°C and the pressure at 0.6±0.05MPa, react for 75 hours, after reacting thoroughly, perform quenching in 30kg(5eq) of dilute hydrochloric acid having a concentration of 10%, and perform suction filtration and drying to the system to obtain a target product, i.e. a crude product of sapropterin dihydrochloride 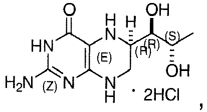 recrystallize and purify the crude product by 17L (10ml/g) of butanone at 15±5°C to obtain 0.6 kg of a pure product, with a yield of 43%, a purity of 98.4% and an enantiomeric excess of 98.9%.

Thus, it can be seen that synthesis of a sapropterin dihydrochloride compound disclosed in a method of the present invention can obtain a target product with a high purity, a high enantiomeric excess, and a high yield. The synthesis method uses readily-available raw materials, significantly reduces a synthesis route of sapropterin dihydrochloride. The technological conditions are stable, and there is less pollution in the whole operation process, hence providing an effective scheme for mass industrial production of sapropterin dihydrochloride.

## Claims

1. A method for synthesizing sapropterin dihydrochloride, **characterized in that** it comprises the following steps:
Step 1: a compound 1 of the formula 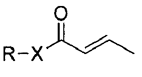 is subjected to epoxidation to generate a compound 2 of the formula 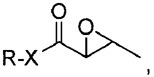 wherein X=NH or O, R=C1-C6 alkane or benzyl;
Step 2: in the presence of acetone, a Lewis acid, an inorganic base liquid, the compound 2 of the formula 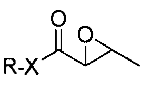 reacts to generate a compound 3 of the formula 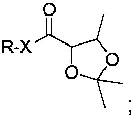
Step 3: the compound 3 of the formula 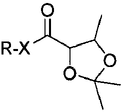 reacts in an alkaline solution, a polar solvent is used to dissolve a filter cake obtained through centrifugation, then a resolving reagent is added to perform resolution to obtain a compound 4 of the formula 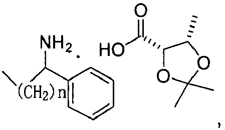 wherein n=0,1;
Step 4: dissolving the compound 4 of the formula 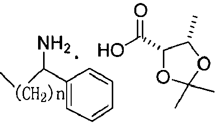 in an ether solvent, and performing separation in acidic conditions to obtain an organic phase, and adding N,N-diisopropylethylamine to the organic phase to obtain a compound 5 of the formula 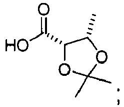
Step 5: using the compound 5 of the formula 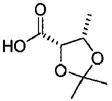 as a raw material to synthesize a compound 6 of the formula 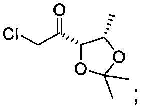
Step 6: reacting the compound 6 of the formula 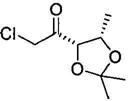 with a trinitride to generate a compound 7 of the formula 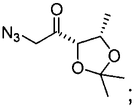
Step 7: subjecting the compound 7 of the formula 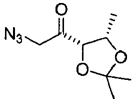 to hydrogenation to obtain a compound 8 of the formula 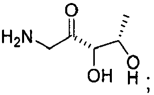
Step 8: reacting the compound 8 of the formula 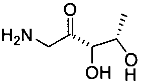 and a compound A of the formula 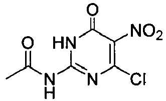 to generate a compound 9 of the formula 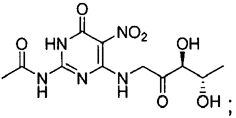
Step 9: subjecting the compound 9 of the formula 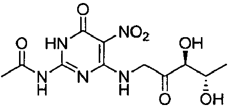 to cyclization to obtain a compound 10 of the formula 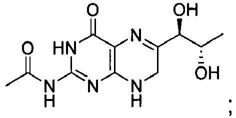
Step 10: adding a catalyst to the compound 10 of the formula 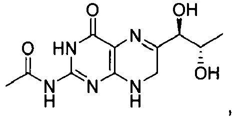 introducing hydrogen to perform a reaction, and then perform quenching in hydrochloric acid having a concentration of 10% to 20% to obtain sapropterin dihydrochloride.

2. The synthesizing method according to claim 1, **characterized in that** it comprises the following steps:
Step 1: in the presence of a polar solvent, adding the compound 1 of the formula R-X 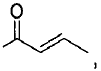 increasing the system temperature to 35°C to 50°C, adding an oxidant, reacting for 2 to 5 hours while preserving the temperature, then adding a strong base aqueous solution having a concentration of 10wt% to 20wt% to the system, while preserving the temperature, reacting the system for 3 to 4 hours after adding the strong base aqueous solution, and performing extraction and concentration to obtain the compound 2 of the formula 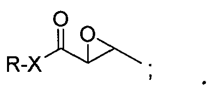
Step 2: adding a Lewis acid in the presence of acetone, controlling the temperature at 10°C to 30°C, adding the compound 2, reacting for 5 to 10 hours while preserving the temperature, adding an inorganic base solution having a concentration of 5wt% to 10wt% to the system, and performing liquid separation, extraction and concentration to the system to obtain the compound 3 of the formula 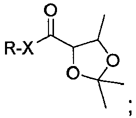
Step 3: adding the compound 3 in the presence of a polar solvent, increasing the temperature to 25°C to 40°C, adding pure water and an alkaline solution, reacting for 3 to 8 hours while preserving the temperature, performing centrifugation, dissolving a filter cake in a polar solvent which is the same as the polar solvent used in the reaction, adding a resolving reagent, preserving the temperature at 15°C to 30°C for 3 to 5 hours, performing centrifugation and drying to obtain the compound 4 of the formula 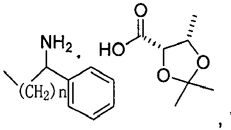 wherein n=0,1;
Step 4: adding the compound 4 in the presence of an ether solvent, then adding an inorganic acid aqueous solution having a concentration of 5% to 10% to the system to regulate the pH to 1 to 3, controlling the temperature at -10°C to 10°C, preserving the temperature for 1 hour, performing liquid separation to obtain an organic phase, adding N,N-diisopropylethylamine to the organic phase, and concentrating the system to obtain the compound 5 of the formula 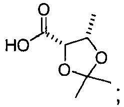
Step 5: adding the compound 5 and N,N-diisopropylethylamine in the presence of an ether solvent, reducing the temperature to -30°C to 0°C, adding a chloroformate, reacting for 1 to 2 hours while preserving the temperature, introducing a diazomethane gas for 1 to 2 hours, adding a hydrochloride ethanol solution, reacting for 1 to 2 hours, adding an alkaline reagent to regulate the pH value to 7 to 9, performing extraction, liquid separation and concentration to obtain the compound 6 of the formula 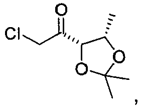
Step 6: adding the compound 6, a trinitride and a catalyst in the presence of a polar solvent, reacting the system at 15°C to 40°C for 20 to 30 hours while preserving the temperature, then performing filtering and concentration to obtain a solution of the compound 7 of the formula 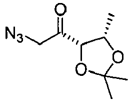 which is used directly in the next step;
Step 7: adding triphenylphosphine and water, or palladium on carbon and hydrogen, or Raney nickel and hydrogen in the presence of an ether solvent, regulating the pH of the system to 1 to 4 with an acid reagent, adding a solution of the compound 7, preserving the temperature for 10°C to 30°C, reacting for 5 to 10 hours, performing suction filtration and concentration to obtain a filtrate containing the compound 8 of the formula 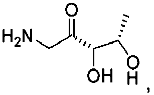 the filtrate being used directly in the next step or a solid of the compound 8 being separated from the filtrate for use in the next step;
Step 8: adding a catalyst, the compound A of the formula 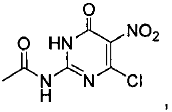 the compound 8, and an alkaline reagent in the presence of an alcoholic solvent and pure water, reacting the system at 30°C to 80°C for 4 to 8 hours while preserving the temperature, adding a buffer solution to regulate the pH of the system to 6 to 8, and filtering the system to obtain the compound 9 of the formula 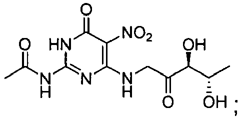
Step 9: adding a catalyst in the presence of the compound 9 and a polar solvent, introducing hydrogen until the pressure of the system is 0.4 to 0.9MPa, controlling the temperature of the system at 15°C to 30°C and the pressure at 0.4 to 0.9MPa, reacting for 18 to 24 hours, filtering the system, and regulating the pH of the system to 11 to 12 with an alkaline reagent to obtain a solution of the compound 10 of the formula 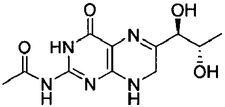 to be used directly in the next step;
Step 10: adding a catalyst in the presence of the solution of the compound 10 obtained in Step 9, introducing hydrogen until the pressure of the system is 0.4 to 0.9MPa, controlling the temperature of the system at 10°C to 30°C, controlling the pressure at 0.4 to 0.9MPa, reacting for 72 to 84 hours, performing quenching in dilute hydrochloric acid having a concentration of 10% to 20% after reacting thoroughly, and performing suction filtration and drying to the system to obtain a sapropterin dihydrochloride crude product.

3. The synthesizing method according to claim 2, **characterized in that** Step 10 further comprises: crystallizing and purifying the sapropterin dihydrochloride crude product with an alcoholic solvent or a ketone solvent at 0°C to 40°C to obtain a sapropterin dihydrochloride pure product.

4. The synthesizing method according to claim 1, **characterized in that** it comprises the following steps:
Step 1: in the presence of a polar solvent, adding the compound 1 of the formula 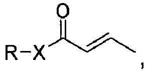 wherein X=NH or O, R=C1 to C6 alkane or benzyl, increasing the system temperature to 35°C to 50°C, adding an oxidant, reacting for 2 to 5 hours while preserving the temperature, then adding a strong base aqueous solution having a concentration of 10% to 20% to the system, while preserving the temperature, reacting the system for 3 to 4 hours after adding the strong base aqueous solution, and performing extraction and concentration to obtain the compound 2 of the formula of 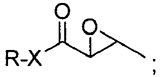 wherein the ratio of the use amount of the compound of formula 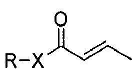 to that of the polar solvent is 1g/5 to 20ml, the molar ratio of the compound of formula R-X 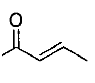 to the oxidant is 1:1 to 3, and the molar ratio of the compound of formula 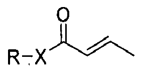 to the strong base is 1:1 to 3;
Step 2: adding a Lewis acid in the presence of acetone, controlling the temperature at 10°C to 30°C, adding the compound 2, reacting for 5 to 10 hours while preserving the temperature, adding an inorganic base solution having a concentration of 5% to 10% to the system, and performing liquid separation, extraction and concentration to the system to obtain the compound 3 of the formula 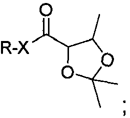 wherein the molar ratio of the compound 2 to acetone is 1:3 to 15; the molar ratio of the compound 2 to the Lewis acid is 1: 0.1 to 1; and the molar ratio of the compound 2 to the inorganic base is 1: 0.5 to 3;
Step 3: adding the compound 3 in the presence of a polar solvent, increasing the temperature to 25°C to 40°C, adding pure water and an alkaline solution, reacting for 3 to 8 hours while preserving the temperature, performing centrifugation, dissolving a filter cake in a polar solvent which is the same as the polar solvent used in the reaction, adding a resolving reagent, preserving the temperature at 15°C to 30°C for 3 to 5 hours, performing centrifugation and drying to obtain the compound 4 of the formula 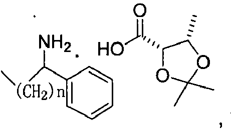 wherein n=0,1;
wherein the ratio of the use amount of the compound 3 to that of the polar solvent used in the reaction is 1 g/3 to 10ml; the molar ratio of the compound 3 to pure water is 1:0.5 to 3; the molar ratio of the compound 3 to an alkaline substance in the alkaline solution is 1:0.5 to 2; the ratio of the use amount of the compound 3 to that of the polar solvent for dissolving the filter cake is 1g/2 to 10ml; and the molar ratio of the compound 3 to the resolving reagent is 1:1 to 5;
Step 4: adding the compound 4 in the presence of an ether solvent, then adding an inorganic acid aqueous solution having a concentration of 5% to 10% to the system to regulate the pH to 1 to 3, controlling the temperature at -10°C to 10°C, performing liquid separation to obtain an organic phase, adding N,N-diisopropylethylamine to the organic phase, and concentrating the system to obtain the compound 5 of the formula 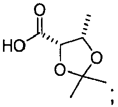 wherein the ratio of the use amount of the compound 4 to that of the ether solvent is 1 g/3 to 10ml and the molar ratio of the compound 4 to N,N-diisopropylethylamine is 1:0.8 to 3;
Step 5: adding the compound 5 and N,N-diisopropylethylamine in the presence of an ether solvent, reducing the temperature to -30°C to 0°C, adding a chloroformate, reacting for 1 to 2 hours while preserving the temperature, introducing a diazomethane gas for 1 to 2 hours, adding a hydrochloride ethanol solution, reacting for 1 to 2 hours, adding an alkaline reagent to regulate the pH value to 7 to 9, performing extraction, liquid separation and concentration to obtain the compound 6 of the formula 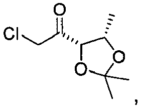 wherein the ratio of the use amount of the compound 5 to that of the ether solvent is 1g/5 to 15ml; the molar ratio of the compound 5 to N,N-diisopropylethylamine is 1:1 to 5; the molar ratio of the compound 5 to the chloroformate is 1:1 to 3; and the molar ratio of the compound 5 to hydrogen chloride in the hydrogen chloride-ethanol solution is 1:1 to 5;
Step 6: adding the compound 6, a trinitride and a catalyst in the presence of a polar solvent, reacting the system at 15°C to 40°C for 20 to 30 hours while preserving the temperature, then performing filtering and concentration to obtain a solution of the compound 7 of the formula 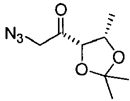 which is used directly in the next step;
wherein the ratio of the use amount of the compound 6 to that of the polar solvent is 1g/5 to 15ml; the molar ratio of the compound 6 to the trinitride is 1:1 to 4; and the molar ratio of the compound 6 to the catalyst is 1:0.05 to 0.8;
Step 7: adding triphenylphosphine and water, or palladium on carbon to and hydrogen, or Raney nickel and hydrogen in the presence of an ether solvent, regulating the pH of the system to 1 to 4 with an acid reagent, adding a solution of the compound 7, preserving the temperature for 10°C to 30°C, reacting for 5 to 10 hours, performing suction filtration and concentration to obtain a filtrate containing the compound 8 of the formula 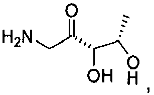 the filtrate being used directly in the next step or a solid of the compound 8 being separated from the filtrate for use in the next step;
wherein the ratio of the use amount of the compound 7 to that of the ether solvent is 1g/5 to 15ml; the molar ratio of the compound 7 to triphenylphosphine is 1:0.1 to 3; the ratio of the use amount of the compound 7 to that of water is 1:0.1 to 3; the mass ratio of the compound 7 to 5% palladium on carbon or 10% palladium on carbon or Raney nickel is 1:0.05 to 0.6; the hydrogen is introduced until the pressure of the system is 0.4 to 0.9MPa;
Step 8: adding a catalyst, the compound A of the formula 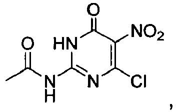 and an alkaline reagent in the presence of an alcoholic solvent and pure water, reacting the system at 30°C to 80°C for 4 to 8 hours while preserving the temperature, adding a buffer solution to regulate the pH of the system to 6 to 8, and filtering the system to obtain the compound 9 of the formula 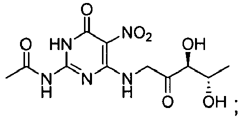 wherein the ratio of the use amount of the compound 8 to that of the alcoholic solvent is 1g/5 to 15ml; the ratio of the use amount of the compound 8 to that of pure water is 1g/1 to 5ml; the molar ratio of the compound 8 to the compound A is 1:1 to 1.5; the molar ratio of compound 8 to the catalyst is 1:0.05 to 0.5; and the molar ratio of the compound 8 to the alkaline reagent is 1:3 to 8;
Step 9: adding a catalyst in the presence of the compound 9 and a polar solvent, introducing hydrogen until the pressure of the system is 0.4 to 0.9MPa, controlling the temperature of the system at 15°C to 30°C and the pressure at 0.4 to 0.9MPa, reacting for 18 to 24 hours, filtering the system, and regulating the pH of the system to 11 to 12 with an alkaline reagent to obtain a solution of the compound 10 of the formula 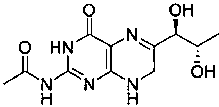 to be used directly in the next step; wherein the ratio of the use amount of the compound 9 to that of the polar solvent is 1g/20 to 50ml and the mass ratio of the compound 9 to the catalyst is 1:0.05 to 0.6;
Step 10: adding a catalyst in the presence of the solution of the compound 10 obtained in Step 9, introducing hydrogen until the pressure of the system is 0.4 to 0.9MPa, controlling the temperature of the system at 10°C to 30°C, controlling the pressure at 0.4 to 0.9MPa, reacting for 72 to 84 hours, performing quenching in dilute hydrochloric acid having a concentration of 10% to 20% after reacting thoroughly, and performing suction filtration and drying to the system to obtain a sapropterin dihydrochloride crude product, and further crystallizing and purifying the sapropterin dihydrochloride crude product with an alcoholic solvent or a ketone solvent at 0°C to 40°C to obtain a sapropterin dihydrochloride pure product,
wherein the mass ratio of the compound 10 to the catalyst is 1:0.05 to 0.6; the molar ratio of the compound 10 to hydrochloric acid is 1:3 to 10; and the ratio of the use amount of the compound 10 to that of the alcoholic solvent or the ketone solvent is 1g/5 to 25ml.

5. The synthesizing method according to claim 4, **characterized in that** in Step 1 the polar solvent is water, methanol, ethanol or isopropanol, preferably water, methanol or ethanol, optimally water; the oxidant is N-bromobutanimide, meta-chloroperoxybenzoic acid, hydrogen peroxide having a concentration of 35% or a toluene solution of tert-butyl hydroperoxide having a concentration of 50%, preferably N-bromobutanimide, meta-chloroperoxybenzoic acid or a toluene solution of tert-butyl hydroperoxide having a concentration of 50%, and optimally N-bromobutanimide; the strong base is sodium hydroxide or potassium hydroxide, preferably sodium hydroxide; the ratio of the use amount of the compound of formula 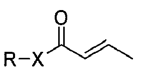 to that of the polar solvent is 1 g/5 to 15ml, preferably 1 g/6 to 12ml; the molar ratio of the compound of formula 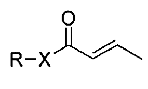 to the oxidant is 1:1 to 2.5, preferably 1:1 to 2; the molar ratio of the compound of formula 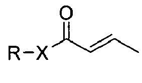 to the strong base is 1:1 to 2.5, preferably 1:1 to 2; preferably, in Step 2, the Lewis acid is aluminium chloride, ferric chloride, zinc chloride, a boron trifluoride diethyl etherate solution having a concentration of 47%, zinc bromide, or lithium chloride, preferably aluminium chloride, the boron trifluoride diethyl etherate solution having a concentration of 47%, zinc bromide or lithium chloride, and optimally the aluminium chloride; the inorganic base is sodium bicarbonate, sodium carbonate, sodium hydroxide, potassium hydroxide, potassium carbonate or potassium bicarbonate, preferably sodium bicarbonate, sodium carbonate, potassium carbonate or potassium bicarbonate, optimally sodium carbonate;
the ratio of the use amount of the compound 2 to that of acetone is 1:5 to 15, preferably 1:5 to 10; the molar ratio of the compound 2 to the Lewis acid is 1:0.1 to 0.8, preferably 1:0.1 to 0.6; the ratio of the use amount of the compound 2 to that of the inorganic base is 1:0.5 to 2.5, preferably 1:0.5 to 1.5;
preferably, in Step 3, the polar solvent is tetrahydrofuran, methanol or ethanol, preferably tetrahydrofuran or methanol, optimally methanol; the resolving reagent is L-α-phenylethylamine or L-α-amphetamine, preferably L-α-phenylethylamine; the alkaline solution is a methanol solution of sodium methoxide having a concentration of 29%, a potassium hydroxide aqueous solution having a concentration of 20% or a sodium hydroxide aqueous solution having a concentration of 20%, preferably the methanol solution of sodium methoxide having a concentration of 29% or the potassium hydroxide aqueous solution having a concentration of 20%, optimally the methanol solution of sodium methoxide having a concentration of 29%; the ratio of the use amount of the compound 3 to that of the polar solvent is 1g/3 to 8ml, preferably 1g/4 to 8ml; the molar ratio of the compound 3 to pure water is 1:0.5 to 1.8, preferably 1:0.5 to 1.5; the molar ratio of the compound 3 to the alkaline substance in the alkaline solution is 1:0.5 to 1.8, preferably 1:0.5 to 1.5; the ratio of the use amount of the compound 3 to that of the polar solvent for dissolving the filter cake is 1g/3 to 8ml, preferably 1g/3 to 7ml; the molar ratio of the compound 3 to the resolving reagent is 1:1 to 4, preferably 1:1 to 3;
preferably, in Step 4, the ether solvent is tetrahydrofuran, 2-methyltetrahydrofuran, methyl tert-butyl ether, 1,4-dioxane or ether, preferably tetrahydrofuran, 2-methyltetrahydrofuran, methyl tert-butyl ether or 1,4-dioxane, optimally 2-methyltetrahydrofuran or 1,4-dioxane; the inorganic acid is sulphuric acid, hydrochloric acid or phosphoric acid, preferably sulfuric acid or hydrochloric acid, optimally sulfuric acid; the ratio of the use amount of the compound 4 to that of the ether solvent is 1g/3 to 8ml, preferably 1g/3 to 6ml; and the molar ratio of the compound 4 to N,N-diisopropylethylamine is 1:0.8 to 2.5, preferably 1:0.8 to 2;
preferably, in Step 5, the ether solvent is tetrahydrofuran, 2-methyltetrahydrofuran, methyl tert-butyl ether, 1,4-dioxane or ether, preferably tetrahydrofuran, 2-methyltetrahydrofuran or methyl tert-butyl ether, optimally tetrahydrofuran or 2-methyltetrahydrofuran; the chloroformate is methyl chloroformate, ethyl chloroformate, or propyl chloroformate, preferably methyl chloroformate or ethyl chloroformate, optimally ethyl chloroformate; the alkaline reagent is triethylamine, sodium carbonate, potassium carbonate, sodium bicarbonate, potassium bicarbonate, sodium hydroxide or potassium hydroxide, preferably triethylamine, sodium carbonate, potassium carbonate, sodium hydroxide or potassium hydroxide, optimally triethylamine; the ratio of the use amount of the compound 5 to that of the ether solvent is 1 g/6 to 12ml, preferably 1g/8 to 12ml; the molar ratio of the compound 5 to N,N-diisopropylethylamine is 1:1.5 to 4, preferably 1:2 to 4; the molar ratio of the compound 5 to the chloroformate is 1:1 to 2.5, preferably 1:1 to 2; and the molar ratio of the compound 5 to hydrogen chloride is 1:1.5 to 4.5, preferably 1:2 to 4;
preferably, in Step 6, the polar solvent is acetonitrile, methanol, ethanol, acetone or tetrahydrofuran, preferably methanol, ethanol or acetone, optimally acetone; the catalyst is sodium iodide or potassium iodide, preferably potassium iodide; the trinitride is sodium azide or azidotrimethylsilane, preferably sodium azide; the ratio of the use amount of the compound 6 to that of the polar solvent is 1g/6 to 12ml, preferably 1g/8 to 12ml; the molar ratio of the compound 6 to the trinitride is 1:1 to 3, preferably 1:1 to 2.5; and the molar ratio of the compound 6 to the catalyst is 1:0.05 to 0.6, preferably 0.1 to 0.5.

6. The synthesizing method according to claim 4, **characterized in that** in Step 7, the ether solvent is tetrahydrofuran, 2-methyltetrahydrofuran, methyl tert-butyl ether, 1,4-dioxane or ether, preferably tetrahydrofuran, 2-methyltetrahydrofuran, methyl tert-butyl ether, or 1,4-dioxane, optimally tetrahydrofuran; the acid reagent is citric acid, p-toluenesulfonic acid, benzenesulfonic acid, formic acid, acetic acid, hydrochloric acid, sulfuric acid or phosphoric acid, preferably citric acid, p-toluenesulfonic acid, benzenesulfonic acid, hydrochloric acid or sulfuric acid, optimally citric acid or hydrochloric acid; the ratio of the use amount of the compound 7 to that of the ether solvent is 1g/5 to 12ml, preferably 1g/6 to 12ml; the molar ratio of the compound 7 to triphenylphosphine is 1:0.6 to 2, preferably 1:0.8 to 2; the ratio of the use amount of the compound 7 to that of water is 1:0.6 to 2, preferably 1:0.8 to 2; the mass ratio of the compound 7 to 5% palladium on carbon or 10% palladium on carbon or Raney nickel is 1:0.05 to 0.4, preferably 1:0.05 to 0.3; hydrogen is introduced until the pressure of the system is 0.5 to 0.8MPa, preferably 0.6 to 0.8MPa.

7. The synthesizing method according to claim 4, **characterized in that** in Step 8, the alcoholic solvent is methanol, ethanol, propanol or isopropanol, preferably methanol, ethanol or isopropanol, optimally isopropanol or ethanol; the catalyst is sodium iodide or potassium iodide, preferably sodium iodide; the alkaline reagent is triethylamine, diisopropylethylamine, diisopropylamine, pyridine, sodium bicarbonate, potassium bicarbonate, sodium carbonate, potassium carbonate or cesium carbonate, preferably triethylamine, pyridine, sodium bicarbonate, potassium bicarbonate, sodium carbonate or potassium carbonate, optimally triethylamine; the buffer solution is a sodium dihydrogen phosphate-disodium hydrogen phosphate aqueous solution, a potassium dihydrogen phosphate-dipotassium hydrogen phosphate aqueous solution or an ammonium formate-ammonia aqueous solution, preferably the sodium dihydrogen phosphate-disodium hydrogen phosphate aqueous solution or the potassium dihydrogen phosphate-dipotassium hydrogen phosphate aqueous solution, optimally the potassium dihydrogen phosphate-dipotassium hydrogen phosphate aqueous solution; the ratio of the use amount of the compound 8 to that of the alcoholic solvent is 1 g/6 to 12ml, preferably 1g/6 to 10ml; the ratio of the use amount of the compound 8 to that of pure water is 1g/1 to 4ml, preferably 1g/1 to 3ml; the molar ratio of the compound 8 to the compound A is 1:1 to 1.4, preferably 1:1 to 1.2; the molar ratio of the compound 8 to the catalyst is 1:0.1 to 0.4, preferably 1:0.1 to 0.3; and the molar ratio of the compound 8 to the alkaline reagent is 1:4 to 7, preferably 1:4 to 6;
preferably, in Step 9, the catalyst is Raney nickel, 5% palladium on carbon, 10% palladium on carbon, platinum dioxide or 20% palladium on carbon, preferably Raney nickel, 5% palladium on carbon or 10% palladium on carbon, optimally Raney nickel; the polar solvent is pure water, methanol or ethanol, preferably pure water and methanol, optimally pure water; the alkaline solution is sodium hydroxide, potassium hydroxide, sodium carbonate or potassium carbonate, preferably sodium hydroxide or sodium carbonate, optimally sodium hydroxide; the ratio of the use amount of the compound 9 to that of the polar solvent is 1g/25 to 45ml, preferably 1g/30 to 40ml and the mass ratio of the compound 9 to the catalyst is 1:0.05 to 0.5, preferably 1:0.1 to 0.4;
preferably, in Step 10, the catalyst is Raney nickel, 5% palladium on carbon, 10% palladium on carbon, platinum dioxide or 20% palladium on carbon, preferably Raney nickel, platinum dioxide or 20% palladium on carbon, optimally 20% palladium on carbon; the alcoholic solvent is methanol, ethanol, isopropanol or n-butanol, preferably methanol, ethanol or isopropanol, optimally methanol; the ketone solvent is acetone or butanone, preferably acetone; the mass ratio of the compound 10 to the catalyst is 1:0.05 to 0.5, preferably 1:0.1 to 0.4; the molar ratio of the compound 10 to hydrochloric acid is 1:4 to 9, preferably 1:5 to 8; and the ratio of the use amount of the compound 10 to that of the alcoholic solvent or the ketone solvent is 1 g/5 to 20ml, preferably 1g/10 to 20ml.

8. A method for preparing (3S,4S)-1-amino-3,4-dihydroxy-2-pentanone, **characterized in that** it comprises the following steps:
Step 1: using a compound 5 of the formula 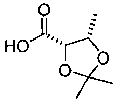 as a raw material to synthesize a compound 6 of the formula 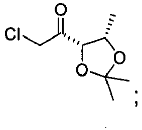
Step 2: reacting the compound 6 of the formula 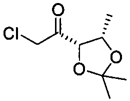 with a trinitride to generate a compound 7 of the formula 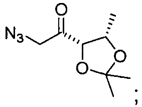
Step 3: subjecting the compound 7 of the formula 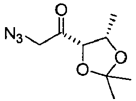 to hydrogenation to obtain a compound 8 of the formula

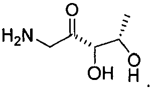

9. The method according to claim 8, **characterized in that** it comprises the following steps:
Step 1: adding the compound 5 and N,N-diisopropylethylamine in the presence of an ether solvent, reducing the temperature to -30°C to 0°C, adding a chloroformate, reacting for 1 to 2 hours while preserving the temperature, introducing a diazomethane gas for 1 to 2 hours, adding a hydrochloride ethanol solution, reacting for 1 to 2 hours, adding an alkaline reagent to regulate the pH value to 7 to 9, performing extraction, liquid separation and concentration to obtain the compound 6 of the formula 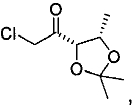
Step 2: adding the compound 6, a trinitride and a catalyst in the presence of a polar solvent, reacting the system at 15°C to 40°C for 20 to 30 hours while preserving the temperature, then performing filtering and concentration to obtain a solution of the compound 7 of the formula 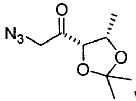 which is used directly in the next step;
Step 3: adding triphenylphosphine and water, or palladium on carbon and hydrogen, or Raney nickel and hydrogen in the presence of an ether solvent, regulating the pH of the system to 1 to 4 with an acid reagent, adding a solution of the compound 7, preserving the temperature at 10°C to 30°C, reacting for 5 to 10 hours, performing suction filtration and concentration to obtain a filtrate containing the compound 8 of the formula 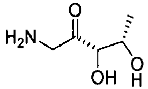 which is used directly in the next step or a solid is separated for use in the next step.

10. The method according to claim 9, **characterized in that** in Step 1, the ratio of the use amount of the compound 5 to that of the ether solvent is 1 g/5 to 15ml; the molar ratio of the compound 5 to N,N-diisopropylethylamine is 1:1 to 5; the molar ratio of the compound 5 to the chloroformate is 1:1 to 3; and the molar ratio of the compound 5 to hydrogen chloride is 1:1 to 5.

11. The method according to claim 10, **characterized in that** in Step 1, the ether solvent is tetrahydrofuran, 2-methyltetrahydrofuran, methyl tert-butyl ether, 1,4-dioxane or ether; the chloroformate is methyl chloroformate, ethyl chloroformate, or propyl chloroformate; the alkaline reagent is triethylamine, sodium carbonate, potassium carbonate, sodium bicarbonate, potassium bicarbonate, sodium hydroxide or potassium hydroxide; the ratio of the use amount of the compound 5 to that of the ether solvent is 1g/6 to 12ml, preferably 1 g/8 to 12ml; the molar ratio of the compound 5 to N,N-diisopropylethylamine is 1:1.5 to 4, preferably 1:2 to 4; the molar ratio of the compound 5 to the chloroformate is 1:1 to 2.5, preferably 1:1 to 2; and the molar ratio of the compound 5 to hydrogen chloride is 1:1.5 to 4.5, preferably 1:2 to 4.

12. The method according to claim 9, **characterized in that** in Step 2, the ratio of the use amount of the compound 6 to that of the polar solvent is 1 g/5 to 15ml; the molar ratio of the compound 6 to the trinitride is 1:1 to 4; and the molar ratio of the compound 6 to the catalyst is 1:0.05 to 0.8.

13. The method according to claim 12, **characterized in that** the polar solvent is acetonitrile, methanol, ethanol, acetone or tetrahydrofuran; the catalyst is sodium iodide or potassium iodide; the trinitride is sodium azide or azidotrimethylsilane; the ratio of the use amount of the compound 6 to that of the polar solvent is 1 g/6 to 12ml, preferably 1 g/8 to 12ml; the molar ratio of the compound 6 to the trinitride is 1:1 to 3, preferably 1:1 to 2.5; and the molar ratio of the compound 6 to the catalyst is 1:0.05 to 0.6, preferably 0.1 to 0.5.

14. The method according to claim 9, **characterized in that** in Step 3, the ratio of the use amount of the compound 7 to that of the ether solvent is 1g/5 to 15ml; the molar ratio of the compound 7 to triphenylphosphine is 1:0.1 to 3; the ratio of the use amount of the compound 7 to that of water is 1:0.1 to 3; the mass ratio of the compound 7 to 5% palladium on carbon or 10% palladium on carbon or Raney nickel is 1:0.05 to 0.6; introducing hydrogen until the pressure of the system is 0.4 to 0.9MPa.

15. The method according to claim 14, **characterized in that** the ether solvent is tetrahydrofuran, 2-methyltetrahydrofuran, methyl tert-butyl ether, 1,4-dioxane or ether; the acid reagent is citric acid, p-toluenesulfonic acid, benzenesulfonic acid, formic acid, acetic acid, hydrochloric acid, sulfuric acid or phosphoric acid; the ratio of the use amount of the compound 7 to that of the ether solvent is 1 g/5 to 12ml, preferably 1 g/6 to 12ml; the molar ratio of the compound 7 to triphenylphosphine is 1:0.6 to 2, preferably 1:0.8 to 2; the ratio of the use amount of the compound 7 to that of water is 1:0.6 to 2, preferably 1:0.8 to 2; the mass ratio of the compound 7 to 5% palladium on carbon or 10% palladium on carbon or Raney nickel is 1:0.05 to 0.4, preferably 1:0.05 to 0.3; introduce hydrogen until the pressure of the system is 0.5 to 0.8MPa, preferably 0.6 to 0.8MPa.

## Patentansprüche

1. Verfahren zum Synthetisieren von Sapropterin-dihydrochlorid, **dadurch gekennzeichnet, dass** es die folgenden Schritte umfasst:
Schritt 1: Eine Verbindung 1 der Formel 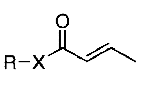 wird einer Epoxidierung unterworfen, um eine Verbindung 2 der Formel 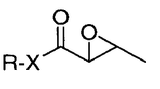 zu erzeugen, wobei X=NH oder O, R=C1-C6-Alkan oder Benzyl;
Schritt 2: In Gegenwart von Aceton, einer Lewis-Säure, einer anorganischen Basenflüssigkeit reagiert die Verbindung 2 der Formel 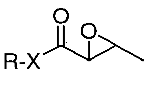 , um eine Verbindung 3 der Formel 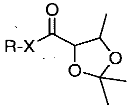 zu erzeugen;
Schritt 3: Die Verbindung 3 der Formel 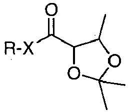 reagiert in einer alkalischen Lösung, ein polares Lösungsmittel wird verwendet, um einen durch Zentrifugation erhaltenen Filterkuchen aufzulösen, anschließend wird ein Trennungsreagenz zugegeben, um eine Trennung bzw. Spaltung durchzuführen, um eine Verbindung 4 der Formel 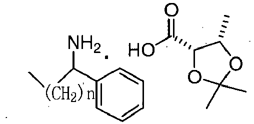 zu erhalten, wobei n=0, 1;
Schritt 4: Auflösen der Verbindung 4 der Formel 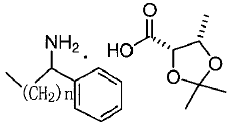 in einem Etherlösungsmittel, und Durchführen einer Trennung unter sauren Bedingungen, um eine organische Phase zu erhalten, und Zugeben von N,N-Diisopropylethylamin zu der organischen Phase, um eine Verbindung 5 der Formel 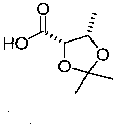 zu erhalten;
Schritt 5: Verwenden der Verbindung 5 der Formel 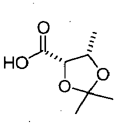 als ein Ausgangsmaterial zum Synthetisieren einer Verbindung 6 der Formel 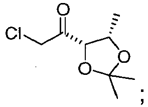
Schritt 6: Umsetzen der Verbindung 6 der Formel 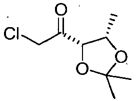 mit einem Trinitrid, um eine Verbindung 7 der Formel 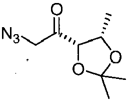 zu erzeugen;
Schritt 7: Unterwerfen der Verbindung 7 der Formel 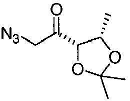 einer Hydrierung, um eine Verbindung 8 der Formel 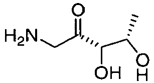 zu erhalten;
Schritt 8: Umsetzen der Verbindung 8 der Formel 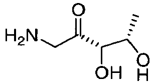 und einer Verbindung A der Formel 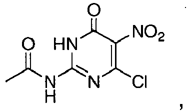 um eine Verbindung 9 der Formel 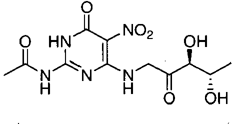 zu erzeugen;
Schritt 9: Unterwerfen der Verbindung 9 der Formel 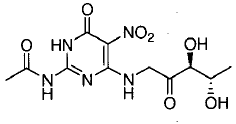 einer Cyclisierung, um eine Verbindung 10 der Formel 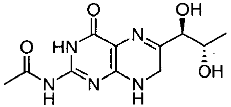 zu erhalten.
Schritt 10: Zugeben eines Katalysators zu der Verbindung 10 der Formel 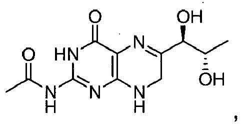 Einführen von Wasserstoff, um eine Reaktion durchzuführen, und anschließend Durchführen eines Quenchens in Chlorwasserstoffsäure mit einer Konzentration von 10 % bis 20 %, um Sapropterin-dihydrochlorid zu erhalten.

2. Syntheseverfahren gemäß Anspruch 1, **dadurch gekennzeichnet, dass** es die folgenden Schritte umfasst:
Schritt 1: Zugeben der Verbindung 1 der Formel 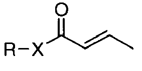 in Gegenwart eines polaren Lösungsmittels, Erhöhen der Systemtemperatur auf 35°C bis 50°C, Zugeben eines Oxidationsmittels, Umsetzen für 2 bis 5 Stunden, während die Temperatur beibehalten wird, anschließend Zugeben einer wässrigen Lösung einer starken Base mit einer Konzentration von 10 Gew.-% bis 20 Gew.-% zu dem System, während die Temperatur beibehalten wird, Umsetzen des Systems für 3 bis 4 Stunden nach dem Zugeben der wässrigen Lösung der starken Base, und Durchführen einer Extraktion und Konzentration, um die Verbindung 2 der Formel 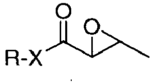 zu erhalten;
Schritt 2: Zugeben einer Lewis-Säure in Gegenwart von Aceton, Regeln der Temperatur auf 10°C bis 30°C, Zugeben der Verbindung 2, Umsetzen für 5 bis 10 Stunden, während die Temperatur beibehalten wird, Zugeben einer Lösung einer anorganischen Base mit einer Konzentration von 5 Gew.-% bis 10 Gew.-% zu dem System, und Durchführen einer Flüssigkeitstrennung, Extraktion und Konzentration an dem System, um die Verbindung 3 der Formel 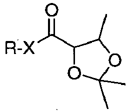 zu erhalten;
Schritt 3: Zugeben der Verbindung 3 in Gegenwart eines polaren Lösungsmittels, Erhöhen der Temperatur auf 25 °C bis 40 °C, Zugeben von reinem Wasser und einer alkalischen Lösung, Umsetzen für 3 bis 8 Stunden, während die Temperatur beibehalten wird, Durchführen einer Zentrifugation, Auflösen eines Filterkuchens in einem polaren Lösungsmittel, welches das gleiche wie das polare Lösungsmittel ist, das in der Reaktion verwendet wird, Zugeben eines Trennungsreagenzes, Beibehalten der Temperatur bei 15°C bis 30°C für 3 bis 5 Stunden, Durchführen einer Zentrifugation und Trocknung, um Verbindung 4 der Formel 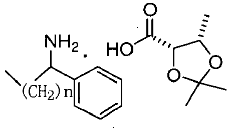 zu erhalten, wobei n=0, 1;
Schritt 4: Zugeben der Verbindung 4 in Gegenwart eines Etherlösungsmittels, anschließend Zugeben einer wässrigen Lösung einer anorganischen Säure mit einer Konzentration von 5 % bis 10 % zu dem System, um den pH auf 1 bis 3 zu regeln, Regeln der Temperatur auf -10 °C bis 10 °C, Beibehalten der Temperatur für 1 Stunde, Durchführen einer Flüssigkeitstrennung, um eine organische Phase zu erhalten, Zugeben von N,N-Diisopropylethylamin zu der organischen Phase, und Konzentrieren des Systems, um die Verbindung 5 der Formel 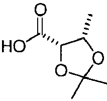 zu erhalten;
Schritt 5: Zugeben von Verbindung 5 und N,N-Diiopropylethylamin in Gegenwart eines Etherlösungsmittels, Verringern der Temperatur auf -30 °C bis 0°C, Zugeben eines Chlorformiats, Umsetzen für 1 bis 2 Stunden, während die Temperatur beibehalten wird, Einführen eines Diazomethangases für 1 bis 2 Stunden, Zugeben einer Hydrochlorid-Ethanol-Lösung, Umsetzen für 1 bis 2 Stunden, Zugeben eines alkalischen Reagenzes zum Einstellen des pH-Werts auf 7 bis 9, Durchführen einer Extraktion, Flüssigkeitstrennung und Konzentration, um die Verbindung 6 der Formel 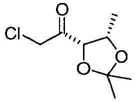 zu erhalten,
Schritt 6: Zugeben von Verbindung 6, einem Trinitrid und einem Katalysator in Gegenwart eines polaren Lösungsmittels, Umsetzen des Systems bei 15°C bis 40°C für 20 bis 30 Stunden, während die Temperatur beibehalten wird, anschließend Durchführen einer Filtration und Konzentration, um eine Lösung der Verbindung 7 der Formel 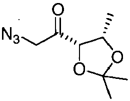 zu erhalten, welche direkt in dem nächsten Schritt verwendet wird;
Schritt 7: Zugeben von Triphenylphosphin und Wasser, oder Palladium auf Kohlenstoff und Wasserstoff, oder Raney-Nickel und Wasserstoff in Gegenwart eines Etherlösungsmittels, Einstellen des pHs des Systems auf 1 bis 4 mit einem Säurereagenz, Zugeben einer Lösung der Verbindung 7, Beibehalten der Temperatur bei 10 °C bis 30 °C, Umsetzen für 5 bis 10 Stunden, Durchführen einer Saugfiltration und Konzentration, um ein Filtrat zu erhalten, das die Verbindung 8 der Formel 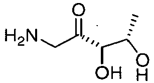 enthält, wobei das Filtrat direkt in dem nächsten Schritt verwendet wird oder ein Feststoff der Verbindung 8 zur Verwendung in dem nächsten Schritt von dem Filtrat abgetrennt wird;
Schritt 8: Zugeben von einem Katalysator, der Verbindung A der Formel 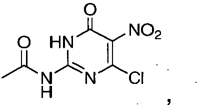 der Verbindung 8 und einem alkalischen Reagenz in Gegenwart von einem alkoholischen Lösungsmittel und reinem Wasser, Umsetzen des Systems bei 30 °C bis 80 °C für 4 bis 8 Stunden, während die Temperatur beibehalten wird, Zugeben einer Pufferlösung, um den pH des Systems auf 6 bis 8 einzustellen, und Filtrieren des Systems, um die Verbindung 9 der Formel 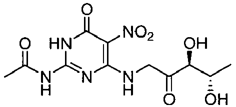 zu erhalten;
Schritt 9: Zugeben eines Katalysators in Gegenwart von Verbindung 9 und einem polaren Lösungsmittel, Einführen von Wasserstoff, bis der Druck des Systems 0,4 bis 0,9 MPa beträgt, Regeln der Temperatur des Systems auf 15 °C bis 30 °C und des Drucks auf 0,4 bis 0,9 MPa, Umsetzen für 18 bis 24 Stunden, Filtrieren des Systems und Einstellen des pHs des Systems auf 11 bis 12 mit einem alkalischen Reagenz, um eine Lösung der Verbindung 10 der Formel 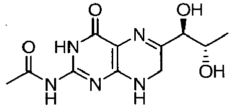 zu erhalten, die direkt in dem nächsten Schritt verwendet werden soll;
Schritt 10: Zugeben eines Katalysators in Gegenwart der Lösung der Verbindung 10, die in Schritt 9 erhalten wurde, Einführen von Wasserstoff, bis der Druck des Systems 0,4 bis 0,9 MPa beträgt, Regeln der Temperatur des Systems auf 10 °C bis 30 °C, Regeln des Drucks auf 0,4 bis 0,9 MPa, Umsetzen für 72 bis 84 Stunden, Durchführen eines Quenchens in verdünnter Chlorwasserstoffsäure mit einer Konzentration von 10 % bis 20 %, nachdem gründlich umgesetzt wurde, und Durchführen einer Saugfiltration und Trocknung an dem System, um ein Sapropterin-dihydrochlorid-Rohprodukt zu erhalten.

3. Syntheseverfahren gemäß Anspruch 2, **dadurch gekennzeichnet, dass** Schritt 10 außerdem umfasst: Kristallisieren und Reinigen des Sapropterin-dihydrochlorid-Rohprodukts mit einem alkoholischen Lösungsmittel oder einem Ketonlösungsmittel bei 0 °C bis 40 °C, um ein Sapropterin-dihydrochlorid-Reinprodukt zu erhalten.

4. Syntheseverfahren gemäß Anspruch 1, **dadurch gekennzeichnet, dass** es die folgenden Schritte umfasst:
Schritt 1: Zugeben der Verbindung 1 der Formel 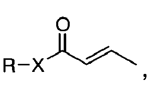 wobei X=NH oder O, R=C1 bis C6-Alkan oder Benzyl, in Gegenwart eines polaren Lösungsmittels, Erhöhen der Systemtemperatur auf 35 °C bis 50 °C, Zugeben eines Oxidationsmittels, Umsetzen für 2 bis 5 Stunden, während die Temperatur beibehalten wird, anschließend Zugeben einer wässrigen Lösung einer starken Base mit einer Konzentration von 10 % bis 20 % zu dem System, während die Temperatur beibehalten wird, Umsetzen des Systems für 3 bis 4 Stunden nach dem Zugeben der wässrigen Lösung der starken Base, und Durchführen einer Extraktion und Konzentration, um die Verbindung 2 der Formel 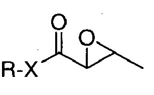 zu erhalten; wobei das Verhältnis der Einsatzmenge der Verbindung der Formel 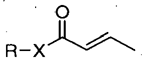 zu der des polaren Lösungsmittels 1 g/5 bis 20 ml beträgt, das Molverhältnis der Verbindung der Formel 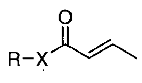 zu dem Oxidationsmittel 1:1 bis 3 beträgt, und das Molverhältnis der Verbindung der Formel 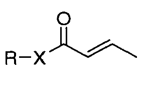 zu der starken Base 1:1 bis 3 beträgt;
Schritt 2: Zugeben einer Lewis-Säure in Gegenwart von Aceton, Regeln der Temperatur auf 10 °C bis 30 °C, Zugeben der Verbindung 2, Umsetzen für 5 bis 10 Stunden, während die Temperatur beibehalten wird, Zugeben einer Lösung einer anorganischen Base mit einer Konzentration von 5 % bis 10 % zu dem System, und Durchführen einer Flüssigkeitstrennung, Extraktion und Konzentration an dem System, um die Verbindung 3 der Formel 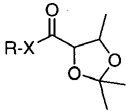 zu erhalten;
wobei das Molverhältnis der Verbindung 2 zu Aceton 1:3 bis 15 beträgt; das Molverhältnis der Verbindung 2 zu der Lewis-Säure 1:0,1 bis 1 beträgt; und das Molverhältnis der Verbindung 2 zu der anorganischen Base 1:0,5 bis 3 beträgt;
Schritt 3: Zugeben der Verbindung 3 in Gegenwart eines polaren Lösungsmittels, Erhöhen der Temperatur auf 25 °C bis 40 °C, Zugeben von reinem Wasser und einer alkalischen Lösung, Umsetzen für 3 bis 8 Stunden, während die Temperatur beibehalten wird, Durchführen einer Zentrifugation, Auflösen eines Filterkuchens in einem polaren Lösungsmittel, welches das gleiche wie das polare Lösungsmittel ist, das in der Reaktion verwendet wird, Zugeben eines Trennungsreagenzes, Beibehalten der Temperatur bei 15 °C bis 30 °C für 3 bis 5 Stunden, Durchführen einer Zentrifugation und Trocknung, um die Verbindung 4 der Formel 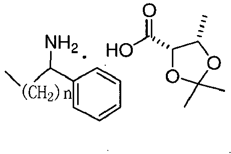 zu erhalten, wobei n=0, 1;
wobei das Verhältnis der Einsatzmenge der Verbindung 3 zu der des polaren Lösungsmittels, das in der Reaktion verwendet wird, 1 g/3 bis 10 ml beträgt; das Molverhältnis der Verbindung 3 zu reinem Wasser 1:0,5 bis 3 beträgt; das Molverhältnis der Verbindung 3 zu einer alkalischen Substanz in der alkalischen Lösung 1:0,5 bis 2 beträgt; das Verhältnis der Einsatzmenge der Verbindung 3 zu der des polaren Lösungsmittels zum Auflösen des Filterkuchens 1 g/2 bis 10 ml beträgt; und das Molverhältnis der Verbindung 3 zu dem Trennungsreagenz 1:1 bis 5 beträgt;
Schritt 4: Zugeben der Verbindung 4 in Gegenwart eines Etherlösungsmittels, anschließend Zugeben einer wässrigen Lösung einer anorganischen Säure mit einer Konzentration von 5 % bis 10 % zu dem System, um den pH auf 1 bis 3 einzustellen, Regeln der Temperatur auf -10 °C bis 10°C, Durchführen einer Flüssigkeitstrennung, um eine organische Phase zu erhalten, Zugeben von N,N-Diisopropylethylamin zu der organischen Phase, und Konzentrieren des Systems, um die Verbindung 5 der Formel 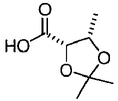 zu erhalten;
wobei das Verhältnis der Einsatzmenge der Verbindung 4 zu der des Etherlösungsmittels 1 g/3 bis 10 ml beträgt und das Molverhältnis der Verbindung 4 zu N,N-Diisopropylethylamin 1:0,8 bis 3 beträgt;
Schritt 5: Zugeben von Verbindung 5 und N,N-Diisopropylethylamin in Gegenwart eines Etherlösungsmittels, Verringern der Temperatur auf -30 °C bis 0 °C, Zugeben eines Chlorformiats, Umsetzen für 1 bis 2 Stunden, während die Temperatur beibehalten wird, Einführen eines Diazomethangases für 1 bis 2 Stunden, Zugeben einer Hydrochlorid-Ethanol-Lösung, Umsetzen für 1 bis 2 Stunden, Zugeben eines alkalischen Reagenzes zum Einstellen des pH-Werts auf 7 bis 9, Durchführen einer Extraktion, Flüssigkeitstrennung und Konzentration, um die Verbindung 6 der Formel 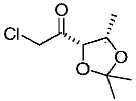 zu erhalten,
wobei das Verhältnis der Einsatzmenge der Verbindung 5 zu der des Etherlösungsmittels 1 g/5 bis 15 ml beträgt; das Molverhältnis der Verbindung 5 zu N,N-Diisopropylethylamin 1:1 bis 5 beträgt; das Molverhältnis der Verbindung 5 zu dem Chlorformiat 1:1 bis 3 beträgt; und das Molverhältnis der Verbindung 5 zu Chlorwasserstoff in der Chlorwasserstoff-Ethanol-Lösung 1:1 bis 5 beträgt;
Schritt 6: Zugeben von Verbindung 6, einem Trinitrid und einem Katalysator in Gegenwart eines polaren Lösungsmittels, Umsetzen des Systems bei 15°C bis 40 °C für 20 bis 30 Stunden, während die Temperatur beibehalten wird, anschließend Durchführen eines Filtrierens und einer Konzentration, um eine Lösung der Verbindung 7 der Formel 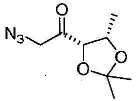 zu erhalten, welche direkt in dem nächsten Schritt verwendet wird;
wobei das Verhältnis der Einsatzmenge der Verbindung 6 zu der des polaren Lösungsmittels 1 g/5 bis 15 ml beträgt; das Molverhältnis der Verbindung 6 zu dem Trinitrid 1:1 bis 4 beträgt; und das Molverhältnis der Verbindung 6 zu dem Katalysator 1:0,05 bis 0,8 beträgt;
Schritt 7: Zugeben von Triphenylphosphin und Wasser, oder Palladium auf Kohlenstoff und Wasserstoff, oder Raney-Nickel und Wasserstoff in Gegenwart eines Etherlösungsmittels, Einstellen des pHs des Systems auf 1 bis 4 mit einem Säurereagenz, Zugeben einer Lösung der Verbindung 7, Beibehalten der Temperatur bei 10 °C bis 30 °C, Umsetzen für 5 bis 10 Stunden, Durchführen einer Saugfiltration und Konzentration, um ein Filtrat zu erhalten, das die Verbindung 8 der Formel 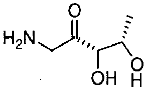 enthält, wobei das Filtrat direkt in dem nächsten Schritt verwendet wird oder ein Feststoff der Verbindung 8 zur Verwendung in dem nächsten Schritt von dem Filtrat abgetrennt wird;
wobei das Verhältnis der Einsatzmenge der Verbindung 7 zu der des Etherlösungsmittels 1 g/5 bis 15 ml beträgt; das Molverhältnis der Verbindung 7 zu Triphenylphosphin 1:0,1 bis 3 beträgt; das Verhältnis der Einsatzmenge der Verbindung 7 zu der von Wasser 1:0,1 bis 3 beträgt, das Massenverhältnis der Verbindung 7 zu 5 % Palladium auf Kohlenstoff oder 10 % Palladium auf Kohlenstoff oder Raney-Nickel 1:0,05 bis 0,6 beträgt; der Wasserstoff eingeführt wird, bis der Druck des Systems 0,4 bis 0,9 MPa beträgt;
Schritt 8: Zugeben von einem Katalysator, der Verbindung A der Formel 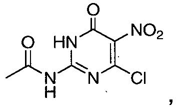 und einem alkalischen Reagenz in Gegenwart von einem alkoholischen Lösungsmittel und reinem Wasser, Umsetzen des Systems bei 30 °C bis 80 °C für 4 bis 8 Stunden, während die Temperatur beibehalten wird, Zugeben einer Pufferlösung, um den pH des Systems auf 6 bis 8 einzustellen, und Filtrieren des Systems, um die Verbindung 9 der Formel 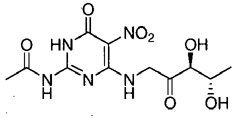 zu erhalten;
wobei das Verhältnis der Einsatzmenge der Verbindung 8 zu der des alkoholischen Lösungsmittels 1 g/5 bis 15 ml beträgt; das Verhältnis der Einsatzmenge der Verbindung 8 zu der von reinem Wasser 1 g/1 bis 5 ml beträgt; das Molverhältnis der Verbindung 8 zu der Verbindung A 1:1 bis 1,5 beträgt, das Molverhältnis von Verbindung 8 zu dem Katalysator 1:0,05 bis 0,5 beträgt; und das Molverhältnis der Verbindung 8 zu dem alkalischen Reagenz 1:3 bis 8 beträgt;
Schritt 9: Zugeben eines Katalysators in Gegenwart von Verbindung 9 und einem polaren Lösungsmittel, Einführen von Wasserstoff, bis der Druck des Systems 0,4 bis 0,9 MPa beträgt, Regeln der Temperatur des Systems auf 15 °C bis 30 °C und des Drucks auf 0,4 bis 0,9 MPa, Umsetzen für 18 bis 24 Stunden, Filtrieren des Systems, und Einstellen des pHs des Systems auf 11 bis 12 mit einem alkalischen Reagenz, um eine Lösung der Verbindung 10 der Formel 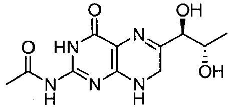 zu erhalten, die direkt in dem nächsten Schritt verwendet werden soll;
wobei das Verhältnis der Einsatzmenge von Verbindung 9 zu der des polaren Lösungsmittels 1 g/20 bis 50 ml beträgt und das Massenverhältnis von Verbindung 9 zu dem Katalysator 1:0,05 bis 0,6 beträgt;
Schritt 10: Zugeben eines Katalysators in Gegenwart der Lösung der Verbindung 10, die in Schritt 9 erhalten wurde, Einführen von Wasserstoff, bis der Druck des Systems 0,4 bis 0,9 MPa beträgt, Regeln der Temperatur des Systems auf 10 °C bis 30 °C, Regeln des Drucks auf 0,4 bis 0,9 MPa, Umsetzen für 72 bis 84 Stunden, Durchführen eines Quenchens in verdünnter Chlorwasserstoffsäure mit einer Konzentration von 10 % bis 20 %, nachdem gründlich umgesetzt wurde, und Durchführen einer Saugfiltration und Trocknung an dem System, um ein Sapropterin-dihydrochlorid-Rohprodukt zu erhalten, und ferner Kristallisieren und Reinigen des Sapropterin-dihydrochlorid-Rohprodukts mit einem alkoholischen Lösungsmittel oder einem Ketonlösungsmittel bei 0 °C bis 40 °C, um ein Sapropterin-dihydrochlorid-Reinprodukt zu erhalten,
wobei das Massenverhältnis von Verbindung 10 zu dem Katalysator 1:0,05 bis 0,6 beträgt; das Molverhältnis von Verbindung 10 zu Chlorwasserstoffsäure 1:3 bis 10 beträgt; und das Verhältnis der Einsatzmenge von Verbindung 10 zu der des alkoholischen Lösungsmittels oder des Ketonlösungsmittels 1 g/5 bis 25 ml beträgt.

5. Syntheseverfahren gemäß Anspruch 4, **dadurch gekennzeichnet, dass** in Schritt 1 das polare Lösungsmittel Wasser, Methanol, Ethanol oder Isopropanol, vorzugsweise Wasser, Methanol oder Ethanol, optimalerweise Wasser ist; das Oxidationsmittel N-Brombutanimid, meta-Chlorperoxybenzoesäure, Wasserstoffperoxid mit einer Konzentration von 35 % oder eine Toluollösung von tert-Butyl-hydroperoxid mit einer Konzentration von 50 %, vorzugsweise N-Brombutanimid, meta-Chlorperoxybenzoesäure oder eine Toluollösung von tert-Butyl-hydroperoxid mit einer Konzentration von 50 %, und optimalerweise N-Brombutanimid ist; die starke Base Natriumhydroxid oder Kaliumhydroxid, vorzugsweise Natriumhydroxid ist; das Verhältnis der Einsatzmenge der Verbindung der Formel 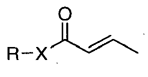 zu der des polaren Lösungsmittels 1 g/5 bis 15 ml, vorzugsweise 1 g/6 bis 12 ml beträgt, das Molverhältnis der Verbindung der Formel 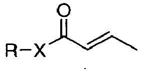 zu dem Oxidationsmittel 1:1 bis 2,5, vorzugsweise 1:1 bis 2 beträgt, das Molverhältnis der Verbindung der Formel 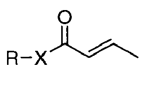 zu der starken Base 1:1 bis 2,5, vorzugsweise 1:1 bis 2 beträgt;
vorzugsweise in Schritt 2 die Lewis-Säure Aluminiumchlorid, Eisen(III)-chlorid, Zinkchlorid, eine Bortrifluorid-Diethyletherat-Lösung mit einer Konzentration von 47 %, Zinkbromid oder Lithiumchlorid, vorzugsweise Aluminiumchlorid, die Bortrifluorid-Diethyletherat-Lösung mit einer Konzentration von 47 %, Zinkbromid oder Lithiumchlorid, und optimalerweise das Aluminiumchlorid ist; die anorganische Base Natriumhydrogencarbonat, Natriumcarbonat, Natriumhydroxid, Kaliumhydroxid, Kaliumcarbonat oder Kaliumhydrogencarbonat, vorzugsweise Natriumhydrogencarbonat, Natriumcarbonat, Kaliumcarbonat oder Kaliumhydrogencarbonat, optimalerweise Natriumcarbonat ist; das Verhältnis der Einsatzmenge der Verbindung 2 zu der von Aceton 1:5 bis 15, vorzugsweise 1:5 bis 10 beträgt; das Molverhältnis der Verbindung 2 zu der Lewis-Säure 1:0,1 bis 0,8, vorzugsweise 1:0,1 bis 0,6 beträgt; das Verhältnis der Einsatzmenge der Verbindung 2 zu der der anorganischen Base 1:0,5 bis 2,5, vorzugsweise 1:0,5 bis 1,5 beträgt;
vorzugsweise in Schritt 3 das polare Lösungsmittel Tetrahydrofuran, Methanol oder Ethanol, vorzugsweise Tetrahydrofuran oder Methanol, optimalerweise Methanol ist; das Trennungsreagenz L-α-Phenylethylamin oder L-α-Amphetamin, vorzugsweise L-α-Phenylethylamin ist; die alkalische Lösung eine Methanollösung von Natriummethoxid mit einer Konzentration von 29 %, eine wässrige Kaliumhydroxidlösung mit einer Konzentration von 20 % oder eine wässrige Natriumhydroxidlösung mit einer Konzentration von 20 %, vorzugsweise die Methanollösung von Natriummethoxid mit einer Konzentration von 29 % oder die wässrige Kaliumhydroxidlösung mit einer Konzentration von 20 %, optimalerweise die Methanollösung von Natriummethoxid mit einer Konzentration von 29 % ist; das Verhältnis der Einsatzmenge der Verbindung 3 zu der des polaren Lösungsmittels 1 g/3 bis 8 ml, vorzugsweise 1 g/4 bis 8 ml beträgt; das Molverhältnis der Verbindung 3 zu reinem Wasser 1:0,5 bis 1,8, vorzugsweise 1:0,5 bis 1,5 beträgt; das Molverhältnis der Verbindung 3 zu der alkalischen Substanz in der alkalischen Lösung 1:0,5 bis 1,8, vorzugsweise 1:0,5 bis 1,5 beträgt; das Verhältnis der Einsatzmenge der Verbindung 3 zu der des polaren Lösungsmittels zum Auflösen des Filterkuchens 1 g/3 bis 8 ml, vorzugsweise 1 g/3 bis 7 ml beträgt; das Molverhältnis der Verbindung 3 zu dem Trennungsreagenz 1:1 bis 4, vorzugsweise 1:1 bis 3 beträgt;
vorzugsweise in Schritt 4 das Etherlösungsmittel Tetrahydrofuran, 2-Methyltetrahydrofuran, Methyl-tert-butylether, 1,4-Dioxan oder Ether, vorzugsweise Tetrahydrofuran, 2-Methyltetrahydrofuran, Methyl-tert-butylether oder 1,4-Dioxan, optimalerweise 2-Methyltetrahydrofuran oder 1,4-Dioxan ist; die anorganische Säure Schwefelsäure, Chlorwasserstoffsäure oder Phosphorsäure, vorzugsweise Schwefelsäure oder Chlorwasserstoffsäure, optimalerweise Schwefelsäure ist; das Verhältnis der Einsatzmenge der Verbindung 4 zu der des Etherlösungsmittels 1 g/3 bis 8 ml, vorzugsweise 1 g/3 bis 6 ml beträgt; und das Molverhältnis der Verbindung 4 zu N,N-Diisopropylethylamin 1:0,8 bis 2,5, vorzugsweise 1:0,8 bis 2 beträgt;
vorzugsweise in Schritt 5 das Etherlösungsmittel Tetrahydrofuran, 2-Methyltetrahydrofuran, Methyl-tert-butylether, 1,4-Dioxan oder Ether, vorzugsweise Tetrahydrofuran, 2-Methyltetrahydrofuran oder Methyl-tert-butylether, optimalerweise Tetrahydrofuran oder 2-Methyltetrahydrofuran ist; das Chlorformiat Methylchlorformiat, Ethylchlorformiat oder Propylchlorformiat, vorzugsweise Methylchlorformiat oder Ethylchlorformiat, optimalerweise Ethylchlorformiat ist; das alkalische Reagenz Triethylamin, Natriumcarbonat, Kaliumcarbonat, Natriumhydrogencarbonat, Kaliumhydrogencarbonat, Natriumhydroxid oder Kaliumhydroxid, vorzugsweise Triethylamin, Natriumcarbonat, Kaliumcarbonat, Natriumhydroxid oder Kaliumhydroxid, optimalerweise Triethylamin ist; das Verhältnis der Einsatzmenge der Verbindung 5 zu der des Etherlösungsmittels 1 g/6 bis 12 ml, vorzugsweise 1 g/8 bis 12 ml beträgt; das Molverhältnis der Verbindung 5 zu N,N-Diisopropylethylamin 1:1,5 bis 4, vorzugsweise 1:2 bis 4 beträgt; das Molverhältnis der Verbindung 5 zu dem Chlorformiat 1:1 bis 2,5, vorzugsweise 1:1 bis 2 beträgt; und das Molverhältnis der Verbindung 5 zu Chlorwasserstoff 1:1,5 bis 4,5, vorzugsweise 1:2 bis 4 beträgt;
vorzugsweise in Schritt 6 das polare Lösungsmittel Acetonitril, Methanol, Ethanol, Aceton oder Tetrahydrofuran, vorzugsweise Methanol, Ethanol oder Aceton, optimalerweise Aceton ist; der Katalysator Natriumiodid oder Kaliumiodid, vorzugsweise Kaliumiodid ist; das Trinitrid Natriumazid oder Azidotrimethylsilan, vorzugsweise Natriumazid ist; das Verhältnis der Einsatzmenge der Verbindung 6 zu der des polaren Lösungsmittels 1 g/6 bis 12 ml, vorzugsweise 1 g/8 bis 12 ml beträgt; das Molverhältnis der Verbindung 6 zu dem Trinitrid 1:1 bis 3, vorzugsweise 1:1 bis 2,5 beträgt; und das Molverhältnis der Verbindung 6 zu dem Katalysator 1:0,05 bis 0,6, vorzugsweise 0,1 bis 0,5 beträgt.

6. Syntheseverfahren gemäß Anspruch 4, **dadurch gekennzeichnet, dass** in Schritt 7 das Etherlösungsmittel Tetrahydrofuran, 2-Methyltetrahydrofuran, Methyl-tert-butylether, 1,4-Dioxan oder Ether, vorzugsweise Tetrahydrofuran, 2-Methyltetrahydrofuran, Methyl-tert-butylether oder 1,4-Dioxan, optimalerweise Tetrahydrofuran ist; das Säurereagenz Citronensäure, p-Toluolsulfonsäure, Benzolsulfonsäure, Ameisensäure, Essigsäure, Chlorwasserstoffsäure, Schwefelsäure oder Phosphorsäure, vorzugsweise Citronensäure, p-Toluolsulfonsäure, Benzolsulfonsäure, Chlorwasserstoffsäure oder Schwefelsäure, optimalerweise Citronensäure oder Chlorwasserstoffsäure ist; das Verhältnis der Einsatzmenge der Verbindung 7 zu der des Etherlösungsmittels 1 g/5 bis 12 ml, vorzugsweise 1 g/6 bis 12 ml beträgt; das Molverhältnis der Verbindung 7 zu Triphenylphosphin 1:0,6 bis 2, vorzugsweise 1:0,8 bis 2 beträgt; das Verhältnis der Einsatzmenge der Verbindung 7 zu der von Wasser 1:0,6 bis 2, vorzugsweise 1:0,8 bis 2 beträgt; das Massenverhältnis der Verbindung 7 zu 5 % Palladium auf Kohlenstoff oder 10 % Palladium auf Kohlenstoff oder Raney-Nickel 1:0,05 bis 0,4, vorzugsweise 1:0,05 bis 0,3 beträgt; Wasserstoff eingeführt wird, bis der Druck des Systems 0,5 bis 0,8 MPa, vorzugsweise 0,6 bis 0,8 MPa beträgt.

7. Syntheseverfahren gemäß Anspruch 4, **dadurch gekennzeichnet, dass** in Schritt 8 das alkoholische Lösungsmittel Methanol, Ethanol, Propanol oder Isopropanol, vorzugsweise Methanol, Ethanol oder Isopropanol, optimalerweise Isopropanol oder Ethanol ist; der Katalysator Natriumiodid oder Kaliumiodid, vorzugsweise Natriumiodid ist; das alkalische Reagenz Triethylamin, Diisopropylethylamin, Diisopropylamin, Pyridin, Natriumhydrogencarbonat, Kaliumhydrogencarbonat, Natriumcarbonat, Kaliumcarbonat oder Cäsiumcarbonat, vorzugsweise Triethylamin, Pyridin, Natriumhydrogencarbonat, Kaliumhydrogencarbonat, Natriumcarbonat oder Kaliumcarbonat, optimalerweise Triethylamin ist; die Pufferlösung eine wässrige Natriumdihydrogenphosphat-Dinatriumhydrogenphosphat-Lösung, eine wässrige Kaliumdihydrogenphosphat-Dikaliumhydrogenphosphat-Lösung oder eine wässrige Ammoniumformiat-Ammoniak-Lösung, vorzugsweise die wässrige Natriumdihydrogenphosphat-Dinatriumhydrogenphosphat-Lösung oder die wässrige Kaliumdihydrogenphosphat-Dikaliumhydrogenphosphat-Lösung, optimalerweise die wässrige Kaliumdihydrogenphosphat-Dikaliumhydrogenphosphat-Lösung ist; das Verhältnis der Einsatzmenge der Verbindung 8 zu der des alkoholischen Lösungsmittels 1 g/6 bis 12 ml, vorzugsweise 1 g/6 bis 10 ml beträgt; das Verhältnis der Einsatzmenge der Verbindung 8 zu der von reinem Wasser 1 g/1 bis 4 ml, vorzugsweise 1 g/1 bis 3 ml beträgt; das Molverhältnis der Verbindung 8 zu der Verbindung A 1:1 bis 1,4, vorzugsweise 1:1 bis 1,2 beträgt; das Molverhältnis der Verbindung 8 zu dem Katalysator 1:0,1 bis 0,4, vorzugsweise 1:0,1 bis 0,3 beträgt; und das Molverhältnis der Verbindung 8 zu dem alkalischen Reagenz 1:4 bis 7, vorzugsweise 1:4 bis 6 beträgt;
vorzugsweise in Schritt 9 der Katalysator Raney-Nickel, 5 % Palladium auf Kohlenstoff, 10% Palladium auf Kohlenstoff, Platindioxid oder 20 % Palladium auf Kohlenstoff, vorzugsweise Raney-Nickel, 5 % Palladium auf Kohlenstoff oder 10 % Palladium auf Kohlenstoff, optimalerweise Raney-Nickel ist; das polare Lösungsmittel reines Wasser, Methanol oder Ethanol, vorzugsweise reines Wasser und Methanol, optimalerweise reines Wasser ist; die alkalische Lösung Natriumhydroxid, Kaliumhydroxid, Natriumcarbonat oder Kaliumcarbonat, vorzugsweise Natriumhydroxid oder Natriumcarbonat, optimalerweise Natriumhydroxid ist; das Verhältnis der Einsatzmenge der Verbindung 9 zu der des polaren Lösungsmittels 1 g/25 bis 45 ml, vorzugsweise 1 g/30 bis 40 ml beträgt und das Massenverhältnis der Verbindung 9 zu dem Katalysator 1:0,05 bis 0,5, vorzugsweise 1:0,1 bis 0,4 beträgt;
vorzugsweise in Schritt 10 der Katalysator Raney-Nickel, 5 % Palladium auf Kohlenstoff, 10 % Palladium auf Kohlenstoff, Platindioxid oder 20 % Palladium auf Kohlenstoff, vorzugsweise Raney-Nickel, Platindioxid oder 20 % Palladium auf Kohlenstoff, optimalerweise 20 % Palladium auf Kohlenstoff ist; das alkoholische Lösungsmittel Methanol, Ethanol, Isopropanol oder n-Butanol, vorzugsweise Methanol, Ethanol oder Isopropanol, optimalerweise Methanol ist; das Ketonlösungsmittel Aceton oder Butanon, vorzugsweise Aceton ist; das Massenverhältnis der Verbindung 10 zu dem Katalysator 1:0,05 bis 0,5, vorzugsweise 1:0,1 bis 0,4 beträgt; das Molverhältnis der Verbindung 10 zu Chlorwasserstoffsäure 1:4 bis 9, vorzugsweise 1:5 bis 8 beträgt; und das Verhältnis der Einsatzmenge der Verbindung 10 zu der des alkoholischen Lösungsmittels oder des Ketonlösungsmittels 1 g/5 bis 20 ml, vorzugsweise 1 g/10 bis 20 ml beträgt.

8. Verfahren zum Herstellen von (3S,4S)-1-Amino-3,4-dihydroxy-2-pentanon, **dadurch gekennzeichnet, dass** es die folgenden Schritte umfasst:
Schritt 1: Verwenden einer Verbindung 5 der Formel 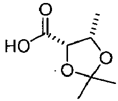 als ein Ausgangsmaterial zum Synthetisieren einer Verbindung 6 der Formel 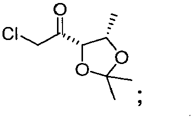
Schritt 2: Umsetzen der Verbindung 6 der Formel 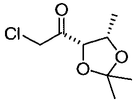 mit einem Trinitrid, um eine Verbindung 7 der Formel 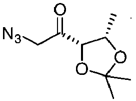 zu erzeugen;
Schritt 3: Unterwerfen der Verbindung 7 der Formel 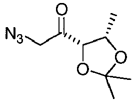 einer Hydrierung, um eine Verbindung 8 der Formel 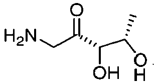 zu erhalten.

9. Verfahren gemäß Anspruch 8, **dadurch gekennzeichnet, dass** es die folgenden Schritte umfasst:
Schritt 1: Zugeben von Verbindung 5 und N,N-Diisopropylethylamin in Gegenwart eines Etherlösungsmittels, Verringern der Temperatur auf -30 °C bis 0 °C, Zugeben eines Chlorformiats, Umsetzen für 1 bis 2 Stunden, während die Temperatur beibehalten wird, Einführen eines Diazomethangases für 1 bis 2 Stunden, Zugeben einer Hydröchlorid-Ethanol-Lösung, Umsetzen für 1 bis 2 Stunden, Zugeben eines alkalischen Reagenzes zum Einstellen des pH-Werts auf 7 bis 9, Durchführen einer Extraktion, Flüssigkeitstrennung und Konzentration, um die Verbindung 6 der Formel 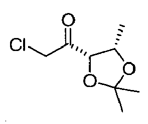 zu erhalten,
Schritt 2: Zugeben von Verbindung 6, einem Trinitrid und einem Katalysator in Gegenwart eines polaren Lösungsmittels, Umsetzen des Systems bei 15 °C bis 40 °C für 20 bis 30 Stunden, während die Temperatur beibehalten wird, anschließend Durchführen einer Filtration und Konzentration, um eine Lösung der Verbindung 7 der Formel 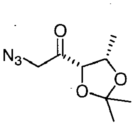 zu erhalten, welche direkt in dem nächsten Schritt verwendet wird.
Schritt 3: Zugeben von Triphenylphosphin und Wasser, oder Palladium auf Kohlenstoff und Wasserstoff, oder Raney-Nickel und Wasserstoff in Gegenwart eines Etherlösungsmittels, Einstellen des pHs des Systems auf 1 bis 4 mit einem Säurereagenz, Zugeben einer Lösung der Verbindung 7, Beibehalten der Temperatur bei 10 °C bis 30 °C, Umsetzen für 5 bis 10 Stunden, Durchführen einer Saugfiltration und Konzentration, um ein Filtrat zu erhalten, das die Verbindung 8 der Formel 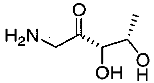 enthält, welches direkt in dem nächsten Schritt verwendet wird oder wobei ein Feststoff zur Verwendung in den nächsten Schritt abgetrennt wird.

10. Verfahren gemäß Anspruch 9, **dadurch gekennzeichnet, dass** in Schritt 1 das Verhältnis der Einsatzmenge der Verbindung 5 zu der des Etherlösungsmittels 1 g/5 bis 15 ml beträgt; das Molverhältnis der Verbindung 5 zu N,N-Diisopropylethylamin 1:1 bis 5 beträgt; das Molverhältnis der Verbindung 5 zu dem Chlorformiat 1:1 bis 3 beträgt; und das Molverhältnis der Verbindung 5 zu Chlorwasserstoff 1:1 bis 5 beträgt.

11. Verfahren gemäß Anspruch 10, **dadurch gekennzeichnet, dass** in Schritt 1 das Etherlösungsmittel Tetrahydrofuran, 2-Methyltetrahydrofuran, Methyl-tert-butylether, 1,4-Dioxan oder Ether ist; das Chlorformiat Methylchlorformiat, Ethylchlorformiat oder Propylchlorformiat ist; das alkalische Reagenz Triethylamin, Natriumcarbonat, Kaliumcarbonat, Natriumhydrogencarbonat, Kaliumhydrogencarbonat, Natriumhydroxid oder Kaliumhydroxid ist; das Verhältnis der Einsatzmenge der Verbindung 5 zu der des Etherlösungsmittels 1 g/6 bis 12 ml, vorzugsweise 1 g/8 bis 12 ml beträgt; das Molverhältnis der Verbindung 5 zu N,N-Diisopropylethylamin 1:1,5 bis 4, vorzugsweise 1:2 bis 4 beträgt; das Molverhältnis der Verbindung 5 zu dem Chlorformiat 1:1 bis 2,5, vorzugsweise 1:1 bis 2 beträgt; und das Molverhältnis der Verbindung 5 zu Chlorwasserstoff 1:1,5 bis 4,5, vorzugsweise 1:2 bis 4 beträgt.

12. Verfahren gemäß Anspruch 9, **dadurch gekennzeichnet, dass** in Schritt 2 das Verhältnis der Einsatzmenge der Verbindung 6 zu der des polaren Lösungsmittels 1 g/5 bis 15 ml beträgt; das Molverhältnis der Verbindung 6 zu dem Trinitrid 1:1 bis 4 beträgt; und das Molverhältnis der Verbindung 6 zu dem Katalysator 1:0,05 bis 0,8 beträgt.

13. Verfahren gemäß Anspruch 12, **dadurch gekennzeichnet, dass** das polare Lösungsmittel Acetonitril, Methanol, Ethanol, Aceton oder Tetrahydrofuran ist; der Katalysator Natriumiodid oder Kaliumiodid ist; das Trinitrid Natriumazid oder Azidotrimethylsilan ist; das Verhältnis der Einsatzmenge der Verbindung 6 zu der des polaren Lösungsmittels 1 g/6 bis 12 ml, vorzugsweise 1 g/8 bis 12 ml beträgt; das Molverhältnis der Verbindung 6 zu dem Trinitrid 1:1 bis 3, vorzugsweise 1:1 bis 2,5 beträgt; und das Molverhältnis der Verbindung 6 zu dem Katalysator 1:0,05 bis 0,6, vorzugsweise 0,1 bis 0,5 beträgt.

14. Verfahren gemäß Anspruch 9, **dadurch gekennzeichnet, dass** in Schritt 3 das Verhältnis der Einsatzmenge der Verbindung 7 zu der des Etherlösungsmittels 1 g/5 bis 15 ml beträgt; das Molverhältnis der Verbindung 7 zu Triphenylphosphin 1:0,1 bis 3 beträgt; das Verhältnis der Einsatzmenge der Verbindung 7 zu der von Wasser 1:0,1 bis 3 beträgt; das Massenverhältnis der Verbindung 7 zu 5 % Palladium auf Kohlenstoff oder 10 % Palladium auf Kohlenstoff oder Raney-Nickel 1:0,05 bis 0,6 beträgt; Wasserstoff eingeführt wird, bis der Druck des Systems 0,4 bis 0,9 MPa beträgt.

15. Verfahren gemäß Anspruch 14, **dadurch gekennzeichnet, dass** das Etherlösungsmittel Tetrahydrofuran, 2-Methyltetrahydrofuran, Methyl-tert-butylether, 1,4-Dioxan oder Ether ist; das Säurereagenz Citronensäure, p-Toluolsulfonsäure, Benzolsulfonsäure, Ameisensäure, Essigsäure, Chlorwasserstoffsäure, Schwefelsäure oder Phosphorsäure ist; das Verhältnis der Einsatzmenge der Verbindung 7 zu der des Etherlösungsmittels 1 g/5 bis 12 ml, vorzugsweise 1 g/6 bis 12 ml beträgt; das Molverhältnis der Verbindung 7 zu Triphenylphosphin 1:0,6 bis 2, vorzugsweise 1:0,8 bis 2 beträgt; das Verhältnis der Einsatzmenge der Verbindung 7 zu der von Wasser 1:0,6 bis 2, vorzugsweise 1:0,8 bis 2 beträgt; das Massenverhältnis der Verbindung 7 zu 5 % Palladium auf Kohlenstoff oder 10 % Palladium auf Kohlenstoff oder Raney-Nickel 1:0,05 bis 0,4, vorzugsweise 1:0,05 bis 0,3 beträgt; Wasserstoff eingeführt wird, bis der Druck des Systems 0,5 bis 0,8 MPa, vorzugsweise 0,6 bis 0,8 MPa beträgt.

## Revendications

1. Procédé de synthèse du dichlorhydrate de saproptérine, **caractérisé en ce qu**'il comprend les étapes suivantes :
Étape 1 : un composé 1 de formule 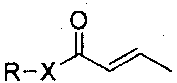 est soumis à une époxydation pour générer un composé 2 de formule 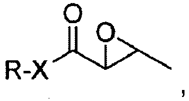 dans laquelle X = NH ou O, R = alcane en C1 à C6 ou benzyle ;
Étape 2 : en présence d'acétone, d'un acide de Lewis, d'un liquide basique inorganique, le composé 2 de formule 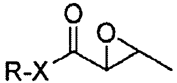 réagit pour donner un composé 3 de formule 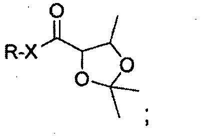
Étape 3 : le composé 3 de formule 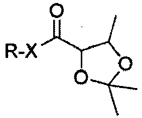 réagit dans une solution alcaline, un solvant polaire est utilisé pour dissoudre un gâteau de filtration obtenu par centrifugation, puis un réactif de résolution est ajouté pour effectuer une résolution pour obtenir un composé 4 de formule 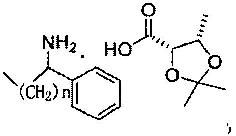 dans laquelle n = 0, 1 ;
Étape 4 : dissolution du composé 4 de formule 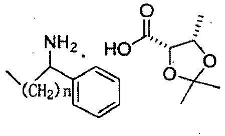 dans un solvant éther, et réalisation d'une séparation dans des conditions acides pour obtenir une phase organique, et ajout de N,N-diisopropyléthylamine à la phase organique pour obtenir un composé 5 de formule 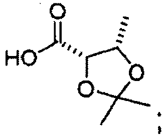
Étape 5 : utilisation du composé 5 de formule 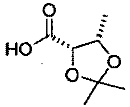 en tant que matière première pour synthétiser un composé 6 de formule 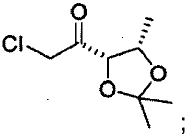
Étape 6 : réaction du composé 6 de formule 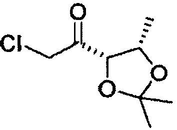 avec un trinitrure pour générer un composé 7 de formule 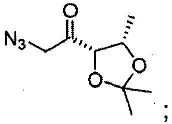
Étape 7 : soumission du composé 7 de formule 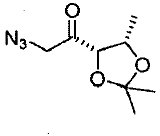 à une hydrogénation pour obtenir un composé 8 de formule 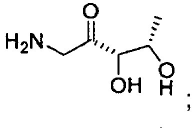
Étape 8 : réaction du composé 8 de formule 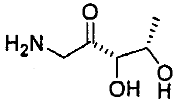 et d'un composé A de formule 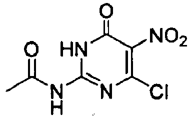 pour générer un composé 9 de formule 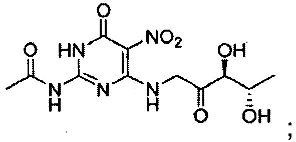
Étape 9 : soumission du composé 9 de formule 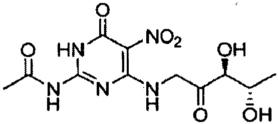 à une cyclisation pour obtenir un composé 10 de formule 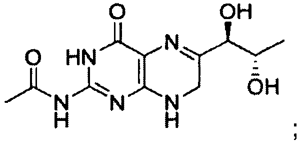
Étape 10 : ajout d'un catalyseur au composé 10 de formule 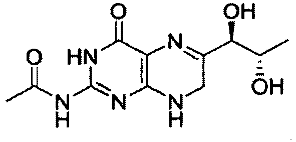 introduction d'hydrogène pour effectuer une réaction, puis extinction dans de l'acide chlorhydrique ayant une concentration de 10 % à 20 % pour obtenir du dichlorhydrate de saproptérine.

2. Procédé de synthèse selon la revendication 1, **caractérisé en ce qu**'il comprend les étapes suivantes :
Étape 1 : en présence d'un solvant polaire, ajout du composé 1 de formule 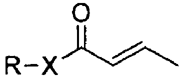 augmentation de la température du système à une valeur de 35 °C à 50 °C, ajout d'un oxydant, réaction pendant 2 à 5 heures tout en maintenant la température, puis ajout d'une solution aqueuse de base forte ayant une concentration de 10 % en poids à 20 % en poids au système, tout en maintenant la température, réaction du système pendant 3 à 4 heures après ajout de la solution aqueuse de base forte, puis réalisation d'une extraction et d'une concentration pour obtenir le composé 2 de formule 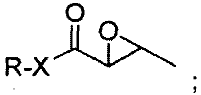
Étape 2 : ajout d'un acide de Lewis en présence d'acétone, régulation de la température à une valeur de 10 °C à 30 °C, ajout du composé 2, réaction pendant 5 à 10 heures tout en maintenant la température, ajout d'une solution de base inorganique ayant une concentration de 5 % en poids à 10 % en poids au système, et application d'une séparation liquide, d'une extraction et d'une concentration au système pour obtenir le composé 3 de formule 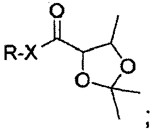
Étape 3 : ajout du composé 3 en présence d'un solvant polaire, augmentation de la température à une valeur de 25 °C à 40 °C, ajout d'eau pure et d'une solution alcaline, réaction pendant 3 à 8 heures tout en maintenant la température, réalisation d'une centrifugation, dissolution d'un gâteau de filtration dans un solvant polaire qui est le même que le solvant polaire utilisé dans la réaction, ajout d'un réactif de résolution, maintien de la température à une valeur de 15 °C à 30 °C pendant 3 à 5 heures, réalisation d'une centrifugation et d'un séchage pour obtenir le composé 4 de formule 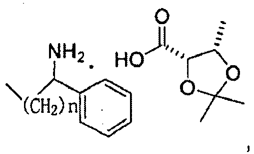 dans laquelle n = 0, 1 ;
Étape 4 : ajout du composé 4 en présence d'un solvant éther, puis ajout d'une solution aqueuse d'acide inorganique ayant une concentration de 5 % à 10 % au système pour ajuster le pH de 1 à 3, régulation de la température à une valeur de -10 °C à 10 °C, maintien de la température pendant 1 heure, réalisation d'une séparation liquide pour obtenir une phase organique, ajout de N,N-diisopropyléthylamine à la phase organique, et concentration du système pour obtenir le composé 5 de formule 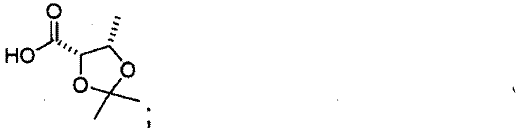
Étape 5 : ajout du composé 5 et de N,N-diisopropyléthylamine en présence d'un solvant éther, réduction de la température à une valeur de -30 °C à 0 °C, ajout d'un chloroformiate, réaction pendant 1 à 2 heures tout en maintenant la température, introduction d'un gaz diazométhane pendant 1 à 2 heures, ajout d'une solution chlorure d'hydrogène éthanol, réaction pendant 1 à 2 heures, ajout d'un réactif alcalin pour ajuster le pH à une valeur de 7 à 9, réalisation d'une extraction, d'une séparation liquide et d'une concentration pour obtenir le composé 6 de formule 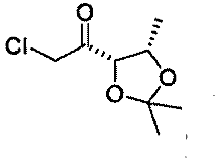
Étape 6 : ajout du composé 6, d'un trinitrure et d'un catalyseur en présence d'un solvant polaire, réaction du système à une température de 15 °C à 40 °C pendant 20 à 30 heures tout en maintenant la température, puis filtration et concentration pour obtenir une solution du composé 7 de formule 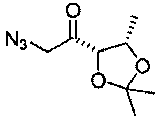 qui est utilisée directement à l'étape suivante ;
Étape 7 : ajout de triphénylphosphine et d'eau, ou de palladium sur carbone et d'hydrogène, ou de nickel de Raney et d'hydrogène en présence d'un solvant éther, ajustement du pH du système de 1 à 4 avec un réactif acide, ajout d'une solution du composé 7, maintien de la température à une valeur de 10 °C à 30 °C, réaction pendant 5 à 10 heures, réalisation d'une filtration par aspiration et d'une concentration pour obtenir un filtrat contenant le composé 8 de formule 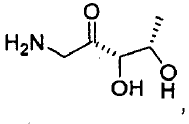 le filtrat étant utilisé directement lors de l'étape suivante ou un solide du composant 8 étant séparé du filtrat pour être utilisé à l'étape suivante ;
Étape 8 : ajout d'un catalyseur, du composé A de formule 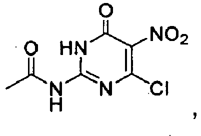 du composé 8 et d'un réactif alcalin en présence d'un solvant alcoolique et d'eau pure, réaction du système à une température de 30 °C à 80 °C pendant 4 à 8 heures tout en maintenant la température, ajout d'une solution tampon pour ajuster le pH du système de 6 à 8, et filtration du système pour obtenir le composé 9 de formule 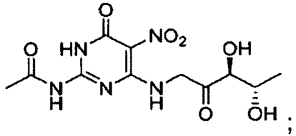
Étape 9 : ajout d'un catalyseur en présence du composé 9 et d'un solvant polaire, introduction d'hydrogène jusqu'à ce que la pression du système soit de 0,4 à 0,9 MPa, régulation de la température du système à une valeur de 15 °C à 30 °C et de la pression à une valeur de 0,4 à 0,9 MPa, réaction pendant 18 à 24 heures, filtrage du système et ajustement du pH de 11 à 12 avec un réactif alcalin pour obtenir une solution du composé 10 de formule 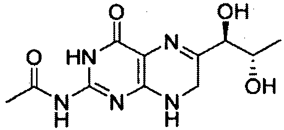 destinée à être utilisée directement à l'étape suivante ;
Étape 10 : ajout d'un catalyseur en présence de la solution du composé 10 obtenue à l'étape 9, introduction d'hydrogène jusqu'à ce que la pression du système soit de 0,4 à 0,9 MPa, régulation de la température du système à une valeur de 10 °C à 30 °C, régulation de la pression à une valeur de 0,4 à 0,9 MPa, réaction pendant 72 à 84 heures, extinction dans de l'acide chlorhydrique dilué ayant une concentration de 10 % à 20 % après réaction complète, et application d'un filtrage par aspiration et d'un séchage au système pour obtenir un produit brut de dichlorhydrate de saproptérine.

3. Procédé de synthèse selon la revendication 2, **caractérisé en ce que** l'étape 10 comprend en outre : la cristallisation et la purification du produit brut de dichlorhydrate de saproptérine avec un solvant alcoolique ou un solvant cétone à une température de 0 °C à 40 °C pour obtenir un produit pur de dichlorhydrate de saproptérine.

4. Procédé de synthèse selon la revendication 1, **caractérisé en ce qu**'il comprend les étapes suivantes :
Étape 1 : en présence d'un solvant polaire, ajout du composé 1 de formule 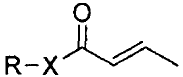 où X = NH ou O, R = alcane en C1 à C6 ou benzyle, augmentation de la température du système à une valeur de 35 °C à 50 °C, ajout d'un oxydant, réaction pendant 2 à 5 heures tout en maintenant la température, puis ajout d'une solution aqueuse de base forte ayant une concentration de 10 % à 20 % dans le système, tout en maintenant la température, réaction du système pendant 3 à 4 heures après ajout de la solution aqueuse de base forte, et réalisation d'une extraction et d'une concentration pour obtenir le composé 2 de formule 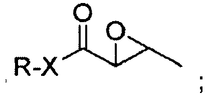 le rapport de la quantité utilisée du composé de formule 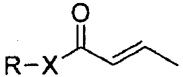 sur celle du solvant polaire étant de 1 g pour 5 à 20 ml, le rapport molaire du composé de formule 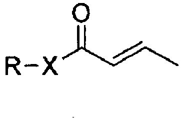 sur l'oxydant allant de 1/1 à 3, et le rapport molaire du composé de formule 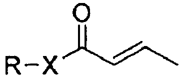 sur la base forte allant de 1/1 à 3 ;
Étape 2 : ajout d'un acide de Lewis en présence d'acétone, régulation de la température à une valeur de 10 °C à 30 °C, ajout du composé 2, réaction pendant 5 à 10 heures tout en maintenant la température, ajout d'une solution de base inorganique ayant une concentration de 5 % à 10 % au système, et application d'une séparation liquide, d'une extraction et d'une concentration au système pour obtenir le composé 3 de formule 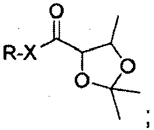 le rapport molaire du composé 2 sur l'acétone allant de 1/3 à 15 ; le rapport molaire du composé 2 sur l'acide de Lewis allant de 1/0,1 à 1 ; et le rapport molaire du composé 2 sur la base inorganique allant de 1/0,5 à 3 ;
Étape 3 : ajout du composé 3 en présence d'un solvant polaire, augmentation de la température à une valeur de 25 °C à 40 °C, ajout d'eau pure et d'une solution alcaline, réaction pendant 3 à 8 heures tout en maintenant la température, réalisation d'une centrifugation, dissolution d'un gâteau de filtration dans un solvant polaire qui est le même que le solvant polaire utilisé dans la réaction, ajout d'un réactif de résolution, maintien de la température à une valeur de 15 °C à 30 °C pendant 3 à 5 heures, réalisation d'une centrifugation et d'un séchage pour obtenir le composé 4 de formule 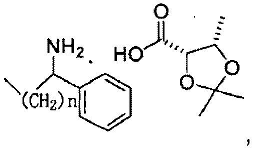 dans laquelle n = 0, 1 ;
le rapport de la quantité utilisée du composé 3 sur celle du solvant polaire utilisé dans la réaction étant de 1 g pour 3 à 10 ml ; le rapport molaire du composé 3 sur l'eau pure allant de 1/0,5 à 3 ; le rapport molaire du composé 3 sur une substance alcaline dans la solution alcaline allant de 1/0,5 à 2 ; le rapport de la quantité utilisée du composé 3 sur celle du solvant polaire utilisé pour dissoudre le gâteau de filtration étant de 1 g pour 2 à 10 ml ; et le rapport molaire du composé 3 sur le réactif de résolution allant de 1/1 à 5 ;
Étape 4 : ajout du composé 4 en présence d'un solvant éther, puis ajout d'une solution aqueuse d'acide inorganique ayant une concentration de 5 % à 10 % au système pour ajuster le pH de 1 à 3, régulation de la température à une valeur de -10 °C à 10 °C, réalisation d'une séparation liquide pour obtenir une phase organique, ajout de N,N-diisopropyléthylamine à la phase organique, et concentration du système pour obtenir le composé 5 de formule 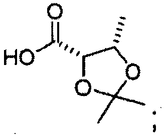 le rapport de la quantité utilisée du composé 4 sur celle du solvant éther étant de 1 g pour 3 à 10 ml et le rapport molaire du composé 4 sur la N,N-diisopropyléthylamine allant de 1/0,8 à 3 ;
Étape 5 : ajout du composé 5 et de N,N-diisopropyléthylamine en présence d'un solvant éther, réduction de la température à une valeur de -30 °C à 0 °C, ajout d'un chloroformiate, réaction pendant 1 à 2 heures tout en maintenant la température, introduction d'un gaz diazométhane pendant 1 à 2 heures, ajout d'une solution chlorhydrate éthanol, réaction pendant 1 à 2 heures, ajout d'un réactif alcalin pour ajuster le pH à une valeur de 7 à 9, réalisation d'une extraction, d'une séparation liquide et d'une concentration pour obtenir le composé 6 de formule 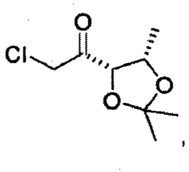 le rapport de la quantité utilisée du composé 5 sur celle du solvant éther étant de 1 g pour 5 à 15 ml ; le rapport molaire du composé 5 sur la N,N-diisopropyléthylamine allant de 1/1 à 5 ; le rapport molaire du composé 5 sur le chloroformiate allant de 1/1 à 3 ; et le rapport molaire du composé 5 sur le chlorhydrate dans la solution chlorhydrate-éthanol allant de 1/1 à 5 ;
Étape 6 : ajout du composé 6, d'un trinitrure et d'un catalyseur en présence d'un solvant polaire, réaction du système à une température de 15 °C à 40 °C pendant 20 à 30 heures tout en maintenant la température, puis réalisation d'une filtration et d'une concentration pour obtenir une solution du composé 7 de formule 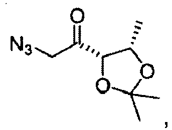 qui est utilisée directement à l'étape suivante ;
le rapport de la quantité utilisée du composé 6 sur celle du solvant polaire étant de 1 g pour 5 à 15 ml ; le rapport molaire du composé 6 sur le trinitrure allant de 1/1 à 4 et le rapport molaire du composé 6 sur le catalyseur allant de 1/0,05 à 0,8 ;
Étape 7 : ajout de triphénylphosphine et d'eau, ou de palladium sur carbone et d'hydrogène, ou de nickel de Raney et d'hydrogène en présence d'un solvant éther, ajustement du pH du système de 1 à 4 avec un réactif acide, ajout d'une solution du composé 7, maintien de la température à une valeur de 10 °C à 30 °C, réaction pendant 5 à 10 heures, réalisation d'une filtration par aspiration et d'une concentration pour obtenir un filtrat contenant le composé 8 de formule 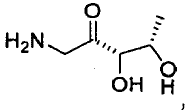 le filtrat étant utilisé directement lors de l'étape suivante ou un solide du composant 8 étant séparé du filtrat pour être utilisé à l'étape suivante ;
le rapport de la quantité utilisée du composé 7 sur celle du solvant éther étant de 1 g pour 5 à 15 ml ; le rapport molaire du composé 7 sur la triphénylphosphine allant de 1/0,1 à 3 ; le rapport de la quantité utilisée du composé 7 sur celle de l'eau étant de 1/0,1 à 3 ; le rapport massique du composé 7 sur le palladium sur carbone à 5 % ou le palladium sur carbone à 10 % ou le nickel de Raney allant de 1/0,05 à 0,6 ; l'hydrogène étant introduit jusqu'à ce que la pression du système soit de 0,4 à 0,9 MPa ;
Étape 8 : ajout d'un catalyseur, du composé A de formule 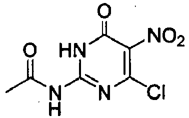 et d'un réactif alcalin en présence d'un solvant alcoolique et d'eau pure, réaction du système à une température de 30 °C à 80 °C pendant 4 à 8 heures tout en maintenant la température, ajout d'une solution tampon pour ajuster le pH du système de 6 à 8, et filtration du système pour obtenir le composé 9 de formule 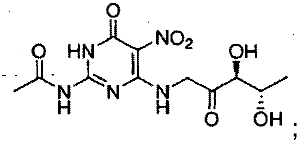 le rapport de la quantité utilisée du composé 8 sur celle du solvant alcoolique étant de 1 g pour 5 à 15 ml ; le rapport de la quantité utilisée de composé 8 sur celle de l'eau pure étant de 1 g pour 1 à 5 ml ; le rapport molaire du composé 8 sur le composé A allant de 1/1 à 5 ; le rapport molaire du composé 8 sur le catalyseur allant de 1/0,05 à 0,5 ; et le rapport molaire du composé 8 sur le réactif alcalin allant de 1/3 à 8 ;
Étape 9 : ajout d'un catalyseur en présence du composé 9 et d'un solvant polaire, introduction d'hydrogène jusqu'à ce que la pression du système soit de 0,4 à 0,9 MPa, régulation de la température du système à une valeur de 15 °C à 30 °C et de la pression à une valeur de 0,4 à 0,9 MPa, réaction pendant 18 à 24 heures, filtrage du système et ajustement du pH de 11 à 12 avec un réactif alcalin pour obtenir une solution du composé 10 de formule 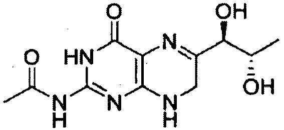 destinée à être utilisée directement à l'étape suivante ;
le rapport de la quantité utilisée du composé 9 sur celle du solvant polaire étant de 1 g pour 20 à 50 ml et le rapport massique du composé 9 sur le catalyseur allant de 1/0,05 à 0,6 ;
Étape 10 : ajout d'un catalyseur en présence de la solution du composé 10 obtenue à l'étape 9, introduction d'hydrogène jusqu'à ce que la pression du système soit de 0,4 à 0,9 MPa, régulation de la température du système à une valeur de 10 °C à 30 °C, régulation de la pression à une valeur de 0,4 à 0,9 MPa, réaction pendant 72 à 84 heures, extinction dans de l'acide chlorhydrique dilué ayant une concentration de 10 % à 20 % après réaction complète, et application d'un filtrage par aspiration et d'un séchage au système pour obtenir un produit brut de dichlorhydrate de saproptérine, puis cristallisation et purification du produit brut de dichlorhydrate de saproptérine avec un solvant alcoolique ou un solvant cétone à une température de 0 °C à 40 °C pour obtenir un produit pur de dichlorhydrate de saproptérine,
le rapport massique du composé 10 sur le catalyseur allant de 1/0,05 à 0,6 ; le rapport molaire du composé 10 sur l'acide chlorhydrique allant de 1/3 à 10 ; et le rapport de la quantité utilisée du composé 10 sur celle du solvant alcoolique ou du solvant cétone étant de 1 g pour 5 à 25 ml.

5. Procédé de synthèse selon la revendication 4, **caractérisé en ce qu**'à l'étape 1 le solvant polaire est de l'eau, du méthanol ou de l'isopropanol, de préférence de l'eau, du méthanol ou de l'éthanol, idéalement de l'eau ; l'oxydant est le N-bromobutanimide, l'acide méta-chloroperoxybenzoïque, du peroxyde d'hydrogène ayant une concentration de 35 % ou une solution dans le toluène de tert-butylhydroperoxyde ayant une concentration de 50 %, de préférence le N-bromobutanimide, l'acide méta-chloroperoxybenzoïque ou une solution dans le toluène de tert-butylhydroperoxyde ayant une concentration de 50 %, et idéalement le N-bromobutanimide ; la base forte est de l'hydroxyde de sodium ou de l'hydroxyde de potassium, de préférence de l'hydroxyde de sodium ; le rapport de la quantité utilisée du composé de formule 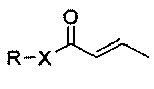 sur celle du solvant polaire est de 1 g pour 5 à 15 ml, de préférence 1 g pour 6 à 12 ml ; le rapport molaire du composé de formule 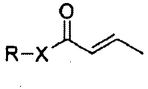 sur l'oxydant va de 1/1 à 2,5, de préférence 1/1 à 2 ; le rapport molaire du composé de formule 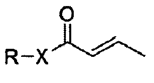 sur la base forte va de 1/1 à 2,5, de préférence 1/1 à 2 ;
de préférence, à l'étape 2, l'acide de Lewis est le chlorure d'aluminium, le chlorure ferrique, le chlorure de zinc, une solution d'éthérate de trifluorure de bore ayant une concentration de 47 %, du bromure de zinc, ou du chlorure de lithium, de préférence du chlorure d'aluminium, la solution de bore trifluorure diéthyle éthérate ayant une concentration de 47 %, du bromure de zinc ou du chlorure de lithium, et idéalement le chlorure d'aluminium ; la base inorganique est du bicarbonate de sodium, du carbonate de sodium, de l'hydroxyde de sodium, de l'hydroxyde de potassium, du carbonate de potassium ou du bicarbonate de potassium, de préférence du bicarbonate de sodium, du carbonate de sodium, du carbonate de potassium ou du bicarbonate de potassium, idéalement du carbonate de sodium ;
le rapport de la quantité utilisée du composé 2 sur celle de l'acétone va de 1/5 à 15, de préférence 1/5 à 10 ; le rapport molaire du composé 2 sur l'acide de Lewis va de 1/0,1 à 0,8, de préférence 1/0,1 à 0,6 ; le rapport de la quantité utilisée du composé 2 sur celle de la base inorganique va de 1/0,5 à 2,5, de préférence 1/0,5 à 1,5 ;
de préférence, à l'étape 3, le solvant polaire est le tétrahydrofurane, le méthanol ou l'éthanol, de préférence le tétrahydrofurane ou le méthanol, idéalement le méthanol ; le réactif de résolution est la L-α-phényléthylamine ou la L-α-amphétamine, de préférence la L-α-phényléthylamine ; la solution alcaline est une solution dans le méthanol de méthoxyde de sodium ayant une concentration de 29 %, une solution aqueuse d'hydroxyde de potassium ayant une concentration de 20 % ou une solution aqueuse d'hydroxyde de sodium ayant une concentration de 20 %, de préférence la solution dans le méthanol de méthoxyde de sodium ayant une concentration de 29 % ou la solution aqueuse d'hydroxyde de potassium ayant une concentration de 20 %, idéalement la solution dans le méthanol de méthoxyde de sodium ayant une concentration de 29 % ; le rapport de la quantité utilisée du composé 3 sur celle du solvant polaire est de 1 g pour 3 à 8 ml, de préférence 1 g pour 4 à 8 ml ; le rapport molaire du composé 3 sur l'eau pure est de 1/0,5 à 1,8, de préférence 1/0,5 à 1,5 ; le rapport molaire du composé 3 sur la substance alcaline dans la solution alcaline va de 1/0,5 à 1,8, de préférence 1/0,5 à 1,5 ; le rapport de la quantité utilisée du composé 3 sur celle du solvant polaire pour dissoudre le gâteau de filtration est de 1 g pour 3 à 8 ml, de préférence 1 g pour 3 à 7 ml ; le rapport molaire du composé 3 sur le réactif de résolution va de 1,1 à 4, de préférence de 1,1 à 3 ;
de préférence, à l'étape 4, le solvant éther est le tétrahydrofurane, le 2-méthyltétrahydrofurane, l'éther de méthyle et de tert-butyle, le 1,4-dioxane ou l'éther, de préférence le tétrahydrofurane, le 2-méthyltétrahydrofurane, l'éther de méthyle et de tert-butyle ou le 1,4-dioxane, idéalement le 2-méthyltétrahydrofurane ou le 1,4-dioxane ; l'acide inorganique est l'acide sulfurique, l'acide chlorhydrique ou l'acide phosphorique, de préférence l'acide sulfurique ou l'acide chlorhydrique, idéalement l'acide sulfurique ; le rapport de la quantité utilisée du composé 4 sur celle du solvant éther est de 1 g pour 3 à 8 ml, de préférence 1 g pour 3 à 6 ml ; et le rapport molaire du composé 4 sur la N,N-diisopropyléthylamine va de 1/0,8 à 2,5, de préférence 1/0,8 à 2 ;
de préférence, à l'étape 5, le solvant éther est le tétrahydrofurane, le 2-méthyltétrahydrofurane, l'éther de méthyle et de tert-butyle, le 1,4-dioxane ou l'éther, de préférence le tétrahydrofurane, le 2-méthyltétrahydrofurane ou l'éther de méthyle et de tert-butyle, idéalement le tétrahydrofurane ou le 2-méthyltétrahydrofurane ; le chloroformiate est le chloroformiate de méthyle, le chloroformiate d'éthyle, ou le chloroformiate de propyle, de préférence le chloroformiate de méthyle ou le chloroformiate d'éthyle, idéalement le chloroformiate d'éthyle ; le réactif alcalin est la triéthylamine, le carbonate de sodium, le carbonate de potassium, le bicarbonate de sodium, le bicarbonate de potassium, l'hydroxyde de sodium ou l'hydroxyde de potassium, de préférence la triéthylamine, le carbonate de sodium, le carbonate de potassium, l'hydroxyde de sodium ou l'hydroxyde de potassium, idéalement la triéthylamine ; le rapport de la quantité utilisée du composé 5 sur celle du solvant éther est de 1 g pour 6 à 12 ml, de préférence 1 g pour 8 à 12 ml ; le rapport molaire du composé 5 sur la N,N-diisopropyléthylamine va de 1/1,5 à 4, de préférence 1/2 à 4 ; le rapport molaire du composé 5 sur le chloroformiate va de 1/1 à 2,5, de préférence 1/1 à 2 ; et le rapport molaire du composé 5 sur le chlorure d'hydrogène va de 1/1,5 à 4,5, de préférence 1/2 à 4 ;
de préférence, à l'étape 6, le solvant polaire est l'acétonitrile, le méthanol, l'éthanol, l'acétone ou le tétrahydrofurane, de préférence le méthanol, l'éthanol ou l'acétone, idéalement l'acétone ; le catalyseur est l'iodure de sodium ou l'iodure de potassium, de préférence l'iodure de potassium ; le trinitrure est l'azoture de sodium ou l'azido(triméthyl)silane, de préférence l'azoture de sodium ; le rapport de la quantité utilisée du composé 6 sur celle du solvant polaire est de 1 g pour 6 à 12 ml, de préférence 1 g pour 8 à 12 ml ; le rapport molaire du composé 6 sur le trinitrure va de 1/1 à 3, de préférence 1/1 à 2,5 ; et le rapport molaire du composé 6 sur le catalyseur va de 1/0,05 à 0,6, de préférence 0,1 à 0,5.

6. Procédé de synthèse selon la revendication 4, **caractérisé en ce qu**'à l'étape 7, le solvant éther est le tétrahydrofurane, le 2-méthyltétrahydrofurane, l'éther de méthyle et de tert-butyle, le 1,4-dioxane ou l'éther, de préférence le tétrahydrofurane, le 2-méthytétrahydrofurane, l'éther de méthyle et de tert-butyle, ou le 1,4-dioxane, idéalement le tétrahydrofurane ; le réactif acide est l'acide citrique, l'acide p-toluènesulfonique, l'acide benzènesulfonique, l'acide formique, l'acide acétique, l'acide chlorhydrique, l'acide sulfurique ou l'acide phosphorique, de préférence l'acide citrique, l'acide p-toluènesulfonique, l'acide benzènesulfonique, l'acide chlorhydrique ou l'acide sulfurique, idéalement l'acide citrique ou l'acide chlorhydrique ; le rapport de la quantité utilisée du composé 7 sur celle du solvant éther est de 1 g pour 5 à 12 ml, de préférence 1 g pour 6 à 12 ml ; le rapport molaire du composé 7 sur la triéthylphosphine va de 0/0,6 à 2, de préférence 1/0,8 à 2 ; le rapport de la quantité utilisée du composé 7 sur celle de l'eau va de 1/0,6 à 2, de préférence 1/0,8 à 2 ; le rapport massique du composé 7 sur le palladium sur carbone à 5 % ou le palladium sur carbone à 10 % ou le nickel de Raney va de 1/0,05 à 0,4, de préférence 1/0,05 à 0,3 ; de l'hydrogène est introduit jusqu'à ce que la pression du système soit de 0,5 à 0,8 MPa, de préférence 0,6 à 0,8 MPa.

7. Procédé de synthèse selon la revendication 4, **caractérisé en ce qu**'à l'étape 8, le solvant alcoolique est le méthanol, l'éthanol, le propanol ou l'isopropanol, de préférence le méthanol, l'éthanol ou l'isopropanol, idéalement l'isopropanol ou l'éthanol ; le catalyseur est l'iodure de sodium ou l'iodure de potassium, de préférence l'iodure de sodium ; le réactif alcalin est la triéthylamine, la diisopropyléthylamine, la diisopropylamine, la pyridine, le bicarbonate de sodium, le bicarbonate de potassium, le carbonate de sodium, le carbonate de potassium, ou le carbonate de césium, de préférence la triéthylamine, la pyridine, le bicarbonate de sodium, le bicarbonate de potassium, le carbonate de sodium ou le carbonate de potassium, idéalement la triéthylamine ; la solution tampon est une solution aqueuse de dihydrogénophosphate de sodium et d'hydrogénophosphate de disodium, une solution aqueuse de dihydrogénophosphate de potassium et d'hydrogénophosphate de dipotassium ou une solution aqueuse de formiate d'ammonium et d'ammoniaque, de préférence la solution aqueuse de dihydrogénophosphate de sodium et d'hydrogénophosphate de disodium ou la solution aqueuse de dihydrogénophosphate de potassium et d'hydrogénophosphate de dipotassium, idéalement la solution aqueuse de dihydrogénophosphate de potassium et d'hydrogénophosphate de dipotassium ; le rapport de la quantité utilisée du composé 8 sur celle du solvant alcoolique est de 1 g pour 6 à 12 ml, de préférence 1 g pour 6 à 10 ml ; le rapport de la quantité utilisée du composé 8 sur celle de l'eau pure est de 1 g pour 1 à 4 ml, de préférence 1 g pour 1 à 3 ml ; le rapport molaire du composé 8 sur le composé A va de 1/1 à 1,4, de préférence 1/1 à 1,2 ; le rapport molaire du composé 8 sur le catalyseur va de 1/0,1 à 0,4, de préférence 1/0,1 à 0,3 ; et le rapport molaire du composé 8 sur le réactif alcalin va de 1/4 à 7, de préférence de 1/4 à 6 ;
de préférence, à l'étape 9, le catalyseur est du nickel de Raney, du palladium sur carbone à 5 %, du palladium sur carbone à 10 %, du dioxyde de platine ou du palladium sur carbone à 20 %, de préférence du nickel de Raney, du palladium sur carbone à 5 % ou du palladium sur carbone à 10 %, idéalement du nickel de Raney ; le solvant polaire est de l'eau pure du méthanol ou de l'éthanol, de préférence de l'eau pure et du méthanol, idéalement de l'eau pure ; la solution alcaline est de l'hydroxyde de sodium, de l'hydroxyde de potassium, du carbonate de sodium ou du carbonate de potassium, de préférence de l'hydroxyde de sodium ou du carbonate de sodium, idéalement de l'hydroxyde de sodium ; le rapport de la quantité utilisée du composé 9 sur celle du solvant polaire est de 1 g pour 25 à 45 ml, de préférence de 1 g pour 30 à 40 ml et le rapport massique du composé 9 sur le catalyseur va de 1/0,05 à 0,5, de préférence 1/0,1 à 0,4 ;
de préférence, à l'étape 10, le catalyseur est du nickel de Raney, du palladium sur carbone à 5 %, du palladium sur carbone à 10 %, du dioxyde de platine ou du palladium sur carbone à 20 %, de préférence du nickel de Raney, du dioxyde de platine ou du palladium sur carbone à 20 %, idéalement du palladium sur carbone à 20 % ; le solvant alcoolique est le méthanol, l'éthanol, l'isopropanol ou le n-butanol, de préférence le méthanol, l'éthanol ou l'isopropanol, idéalement le méthanol ; le solvant cétone est l'acétone ou la butanone, de préférence l'acétone ; le rapport massique du composé 10 sur le catalyseur va de 1/0,05 à 0,5, de préférence 1/0,1 à 0,4 ; le rapport molaire du composé 10 sur l'acide chlorhydrique va de 1/4 à 9, de préférence 1/5 à 8 ; et le rapport de la quantité utilisée du composé 10 sur celle du solvant alcoolique ou du solvant cétone est de 1 g pour 5 à 20 ml, de préférence de 1 g pour 10 à 20 ml.

8. Procédé de préparation de la (3S,4S)-1-amino-3,4-dihydroxy-2-pentanone, **caractérisé en ce qu**'il comprend les étapes suivantes :
Étape 1 : utilisation d'un composé 5 de formule 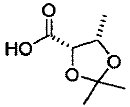 en tant que matière première pour synthétiser un composé 6 de formule 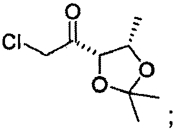
Étape 2 : réaction du composé 6 de formule 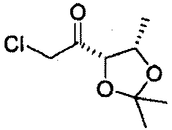 avec un trinitrure pour générer un composé 7 de formule 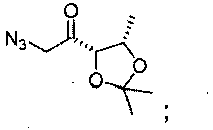
Étape 3 : soumission du composé 7 de formule 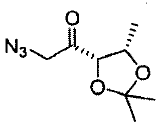 à une hydrogénation pour obtenir un composé 8 de formule

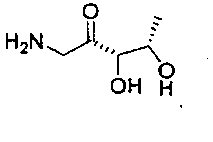

9. Procédé selon la revendication 8, **caractérisé en qu**'il comprend les étapes suivantes :
Étape 1 ; ajout du composé 5 et de N,N-diisopropyléthylamine en présence d'un solvant éther, réduction de la température à une valeur de -30 °C à 0 °C, ajout d'un chloroformiate, réaction pendant 1 à 2 heures tout en maintenant la température, introduction d'un gaz diazométhane pendant 1 à 2 heures, ajout d'une solution chlorure d'hydrogène éthanol, réaction pendant 1 à 2 heures, ajout d'un réactif alcalin pour ajuster le pH à une valeur de 7 à 9, réalisation d'une extraction, d'une séparation liquide et d'une concentration pour obtenir le composé 6 de formule 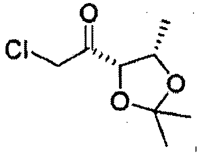
Étape 2 : ajout du composé 6, d'un trinitrure et d'un catalyseur en présence d'un solvant polaire, réaction du système à une température de 15 °C à 40 °C pendant 20 à 30 heures tout en maintenant la température, puis réalisation d'une filtration et d'une concentration pour obtenir une solution du composé 7 de formule 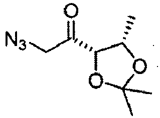 qui est utilisée directement à l'étape suivante ;
Étape 3 : ajout de triphénylphosphine et d'eau, ou de palladium sur carbone et d'hydrogène, ou de nickel de Raney et d'hydrogène en présence d'un solvant éther, ajustement du pH du système de 1 à 4 avec un réactif acide, ajout d'une solution du composé 7, maintien de la température à une valeur de 10 °C à 30 °C, réaction pendant 5 à 10 heures, réalisation d'une filtration par aspiration et d'une concentration pour obtenir un filtrat contenant le composé 8 de formule 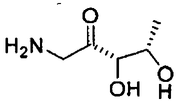 qui est utilisé directement lors de l'étape suivante ou un solide est séparé pour être utilisé à l'étape suivante.

10. Procédé selon la revendication 9, **caractérisé en ce qu**'à l'étape 1, le rapport de la quantité utilisée du composé 5 sur celle de solvant éther est de 1 g pour 5 à 15 ml ; le rapport molaire du composé 5 sur la N,N-diisopropyléthylamine va de 1/1 à 5 ; le rapport molaire du composé 5 sur le chloroformiate va de 1/1 à 3 ; et le rapport molaire du composé 5 sur le chlorure d'hydrogène va de 1/1 à 5.

11. Procédé selon la revendication 10, **caractérisé en ce qu**'à l'étape 1, le solvant éther est le tétrahydrofurane, le 2-méthyltétrathydrofurane, l'éther de méthyle et de tert-butyle, le 1,4-dioxane ou l'éther ; le chloroformiate est le chloroformiate de méthyle, le chloroformiate d'éthyle, ou le chloroformiate de propyle ; le réactif alcalin est la triéthylamine, le carbonate de sodium, le carbonate de potassium, le bicarbonate de sodium, le bicarbonate de potassium, l'hydroxyde de sodium ou l'hydroxyde de potassium ; le rapport de la quantité utilisée du composé 5 sur celle de solvant éther est de 1 g pour 6 à 12 ml, de préférence 1 g pour 8 à 12 ml ; le rapport molaire du composé 5 sur la N,N-diisopropyléthylamine va de 1/1,5 à 4, de préférence 1/2 à 4 ; le rapport molaire du composé 5 sur le chloroformiate va de 1/1 à 2,5, de préférence 1/1 à 2 ; et le rapport molaire du composé 5 sur le chlorure d'hydrogène va de 1/1,5 à 4,5, de préférence 1/2 à 4.

12. Procédé selon la revendication 9, **caractérisé en ce qu'**à l'étape 2, le rapport de la quantité utilisée du composé 6 sur celle du solvant polaire est de 1 g pour 5 à 15 ml ; le rapport molaire du composé 6 sur le trinitrure va de 1/1 à 4 ; et le rapport molaire du composé 6 sur le catalyseur va de 1/0,05 à 0,8.

13. Procédé selon la revendication 12, **caractérisé en ce que** le solvant polaire est l'acétonitrile, l'éthanol, l'acétone ou le tétrahydrofurane ; le catalyseur est l'iodure de sodium ou l'iodure de potassium ; le trinitrure est l'azoture de sodium ou l'azido(triméthyl)silane ; le rapport de la quantité utilisée du composé 6 sur celle du solvant polaire est de 1 g pour 6 à 12 ml, de préférence 1 g pour 8 à 12 ml ; le rapport molaire du composé 6 sur le trinitrure va de 1/1 à 3, de préférence 1/1 à 2,5 ; et le rapport molaire du composé 6 sur le catalyseur va de 1/0,05 à 0,6, de préférence 0,1 à 0,5.

14. Procédé selon la revendication 9, **caractérisé en ce qu**'à l'étape 3, le rapport de la quantité utilisée du composé 7 sur celle du solvant éther est de 1 g pour 5 à 15 ml ; le rapport molaire du composé 7 sur la triphénylphosphine va de 1/0,1 à 3 ; le rapport de la quantité utilisée du composé 7 sur celle de l'eau est de 1/0,1 à 3 ; le rapport massique du composé 7 sur le palladium sur carbone à 5 % ou le palladium sur carbone à 10 % ou le nickel de Raney va de 1/0,05 à 0,6 ; introduction d'hydrogène jusqu'à ce que la pression du système soit de 0,4 à 0,9 MPa.

15. Procédé selon la revendication 14, **caractérisé en ce que** le solvant éther est le tétrahydrofurane, le 2-méthyltétrahydrofurane, l'éther de méthyle et de tert-butyle, le 1,4-dioxane ou l'éther ; le réactif acide est l'acide citrique, l'acide p-toluènesulfonique, l'acide benzènesulfonique, l'acide formique, l'acide acétique, l'acide chlorhydrique, l'acide sulfurique ou l'acide phosphorique ; le rapport de la quantité utilisée du composé 7 sur celle du solvant éther est de 1 g pour 5 à 12 ml, de préférence 1 g pour 6 à 12 ml ; le rapport molaire du composé 7 sur la triphénylphosphine va de 1/0,6 à 2, de préférence 1/0,8 à 2 ; le rapport de la quantité utilisée du composé 7 sur celle de l'eau va de 1/0,6 à 2, de préférence 1/0,8 à 2 ; le rapport massique du composé 7 sur le palladium sur carbone à 5 % ou le palladium sur carbone à 10 % ou le nickel de Raney va de 1/0,05 à 0,4, de préférence 1/0,05 à 0,3 ; introduction d'hydrogène jusqu'à ce que la pression du système soit de 0,5 à 0,8 MPa, de préférence 0,6 à 0,8 MPa.
